# EUROPEAN PATENT SPECIFICATION

(11) **EP 4 245 293 B1**
(45) Date of publication and mention of the grant of the patent: **06.08.2025**
(21) Application number: 22162354.9
(22) Date of filing: 15.03.2022
(51) Int. Cl.: A61K 8/60, A61K 8/41, A61Q 1/10, A61Q 11/00, A61Q 5/10, A61Q 5/12, A61Q 5/02, A61Q 17/04, A61Q 19/10, C11D 7/32

(54) **USE OF SUGAR AMINES AS COMPLEXING AGENTS**
VERWENDUNG VON ZUCKERAMINEN ALS KOMPLEXBILDNER
UTILISATION D'AMINES DE SUCRE EN TANT QU'AGENTS COMPLEXANTS

(43) Date of publication of application: 20.09.2023
(73) Proprietor: Clariant International Ltd, 4132 Muttenz (CH)
(72) Inventor: ZMIRIC, Aleksandra, 20037 Palazzolo Milanese (IT); JOHANNPETER, Wiebke, 61449 Steinbach am Taunus (DE); BRAAK, Svea, 65926 Frankfurt am Main (DE); RAUCH, Gesche, 65926 Frankfurt am Main (DE)
(74) Representative: Clariant Produkte (Deutschland) GmbH

(56) References cited:
- EP-A1- 3 444 325
- EP-A1- 3 939 563
- WO-A1-2014/138141
- WO-A1-2017/060481
- WO-A1-98/46217
- WO-A2-02/30383
- JP-A- 2007 154 081
- KR-A- 20130 053 768

## Description

The present invention relates to the use of a sugar amine or a salt thereof in a cosmetic composition or a home care composition as a complexing agent, preferably as a chelating agent.

Complexing agents, including chelating agents, have proven to be versatile ingredients for various applications. For example, they are frequently used in cosmetics, cleaning products, and on textiles. See e.g. WO 2014/138141 A1 (PROCTER & GAMBLE [US]) 12 September 2014 (2014-09-12) or EP 3 444 325 A1 (PROCTER & GAMBLE [US]) 20 February 2019 (2019-02-20).

Complexing agents, including chelating agents, can bind metals and help stabilize products against the effects of metal contamination. Metals can enter cosmetic formulations or home care formulations in multiple ways, for example from the production equipment and storage containers, or as impurities from ingredients and water. In cosmetic formulations and home care formulations, metals can promote oxidation reactions, impair the foaming properties of surfactants, potentially cause discoloration, or enhance microbial contamination.

EDTA is a traditional chelating agent. However, it is under scrutiny. EDTA slowly biodegrades and accumulates in the environment.

There is an ongoing need for complexing agents, including chelating agents, for various applications, including cosmetic and home care applications.

Consumers have generally become more conscious about the ingredients used in, e.g., cosmetic products or home care products. Among others, they desire sustainable and environmentally friendly ingredients that are derived from natural and renewable sources.

It has now been found that sugar amines (such as N,N-dimethylglucamine) or salts thereof are excellent complexing agents, preferably chelating agents, for a variety of cosmetic and home care applications.

The present invention relates to the use of a sugar amine selected from N,N-dimethylglucamine, N-methylglucamine, N-methylxylamine, N,N-dimethylxylamine, N-methylmaltosamine, N,N-dimethylmaltosamine, N-ethylglucamine, N,N-diethylglucamine, N-methylpropylglucamine, N-2-hydroxyethylglucamine, N,N-methyl 2-hydroxyethylglucamine, N-methylfructosamine, N,N-dimethylfructosamine, N-methylmaltulosamine, N,N-dimethylmaltulosamine, N-methylmaltotriosamine, N,N-dimethylmaltotriosamine, and mixtures thereof, or a salt thereof in a cosmetic composition or a home care composition as a complexing agent, preferably as a chelating agent.

Advantageously, sugar amines (such as N,N-dimethylglucamine) or salts thereof show an excellent performance as a complexing agent, preferably as a chelating agent. Furthermore, sugar amines (such as N,N-dimethylglucamine) are sugar-based, i.e. derived from natural and renewable resources, and thus sustainable and environmentally friendly. Moreover, they are readily biodegradable.

According to the invention, a sugar amine (preferably N,N-dimethylglucamine) or a salt thereof is used in a cosmetic composition or a home care composition as a complexing agent, preferably as a chelating agent.

Preferred sugar amines are selected from N,N-dimethylglucamine, N-methylglucamine, N-methylxylamine, N,N-dimethylxylamine, N-methylmaltosamine, N,N-dimethylmaltosamine, N-ethylglucamine, N,N-diethylglucamine, N-methylpropylglucamine, N-2-hydroxyethylglucamine, N,N-methyl 2-hydroxyethylglucamine, N-methylfructosamine, N,N-dimethylfructosamine, N-methylmaltulosamine, N,N-dimethylmaltulosamine, N-methylmaltotriosamine, N,N-dimethylmaltotriosamine, and mixtures thereof.

More preferred sugar amines are selected from N,N-dimethylglucamine, N-methylglucamine, N-methylxylamine, N-methylmaltosamine, and mixtures thereof. A particularly preferred sugar amine is N,N-dimethylglucamine.

The term "N,N-dimethylglucamine" as used herein refers to a compound of Formula (I):

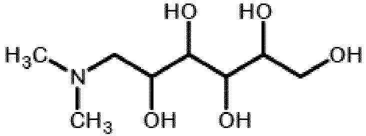

Other names of N,N-dimethylglucamine are dimethylglucamine or 1-deoxy-1-(dimethylamino)-glucitol.

The term "N,N-dimethylglucamine" as used herein encompasses N,N-dimethyl-D-glucamine and N,N-dimethyl-L-glucamine. Other names of N,N-dimethyl-D-glucamine are dimethyl-D-glucamine or 1-deoxy-1-(dimethylamino)-D-glucitol. Other names of N,N-dimethyl-L-glucamine are dimethyl-L-glucamine or 1-deoxy-1-(dimethylamino)-L-glucitol. In a preferred embodiment, the term "N,N-dimethylglucamine" as used herein refers to N,N-dimethyl-D-glucamine.

In a preferred embodiment, a sugar amine is used. In another embodiment, a salt of a sugar amine is used.

In a particularly preferred embodiment, N,N-dimethylglucamine is used. In another particular embodiment, a salt of N,N-dimethylglucamine is used.

Suitable salts of sugar amines are, for example, any salts that sugar amines may form under acidic conditions or in an acidic environment.

Further examples of suitable salts of sugar amines are sugar amines hydrochlorides, sugar amines hydrobromides, acetic acid salts of sugar amines, fatty acid salts of sugar amines, sorbic acid salts of sugar amines, benzoic acid salts of sugar amines, anisic acid salts of sugar amines, or mixtures thereof.

Suitable salts of N,N-dimethylglucamine are, for example, any salts that N,N-dimethylglucamine may form under acidic conditions or in an acidic environment.

Further examples of suitable salts of N,N-dimethylglucamine are N,N-dimethylglucamine hydrochloride, N,N-dimethylglucamine hydrobromide, the acetic acid salt of N,N-dimethylglucamine, fatty acid salts of N,N-dimethylglucamine, the sorbic acid salt of N,N-dimethylglucamine, the benzoic acid salt of N,N-dimethylglucamine, the anisic acid salt of N,N-dimethylglucamine, or mixtures thereof.

Sugar amines are known in the art, e.g. from WO 2017/060491 A1 and WO 2018/035192 A1. Sugar amines are commercially available or can be prepared according to methods known in the art. N,N-dimethylglucamine is commercially available (e.g. as Neutrotain^{™} DMG, Clariant) or can be prepared according to methods known in the art (e.g. as described in US 2021/0114968 A1).

The sugar amine (preferably N,N-dimethylglucamine) or a salt thereof may, for example, be used as such or as a solution in a solvent. Preferred solvents are water or alcohols. More preferred solvents are water, ethanol, isopropanol, ethylene glycol, propylene glycol, glycerin, or mixtures thereof. A particularly preferred solvent is water. In one embodiment, the sugar amine (preferably N,N-dimethylglucamine) or a salt thereof is used as an aqueous solution.

According to the invention, a sugar amine (preferably N,N-dimethylglucamine) or a salt thereof is used as a complexing agent, preferably as a chelating agent.

The term "complexing agent" is known in the art. Preferably, the term "complexing agent" as used herein refers to a chemical species capable of binding with metal ions or other chemical entities in a system through its single or multiple sites. These sites have lone pairs of electrons, which can, for example, be donated to the d orbitals of a metal ion, forming coordination bonds. This results in a complex. For example, a complexing agent may surround a metal ion or may act as a bridge between two metal ions. A complexing agent can, for example, be a molecule or an ion. A complexing agent can have one or more binding sites (in the same molecule).

The term "chelating agent" is known in the art. Preferably, the term "chelating agent" as used herein refers to a chemical species capable of binding with metal ions or other chemical entities in a system through its multiple sites. These sites have lone pairs of electrons, which can, for example, be donated to the d orbitals of a metal ion, forming coordination bonds. This results in a chelate complex. For example, a chelating agent may surround a metal ion or may act as a bridge between two metal ions. A chelating agent can, for example, be a molecule or an ion. A chelating agent can have two or more binding sites in the same molecule.

In a particularly preferred embodiment, the sugar amine (preferably N,N-dimethylglucamine) or salt thereof is used as a complexing agent, preferably as a chelating agent, to bind metal ions.

Preferred metal ions are selected from alkali metal ions, alkaline earth metal ions, aluminum ions, transition metal ions, rare earth metal ions, noble metal ions, and mixtures thereof.

More preferred metal ions are selected from alkaline earth metal ions, aluminum ions, transition metal ions, and mixtures thereof. Even more preferred metal ions are selected from alkaline earth metal ions, transition metal ions, and mixtures thereof.

Particularly preferred metal ions are selected from transition metal ions. Also particularly preferred metal ions are selected from alkaline earth metal ions.

Examples of preferred metal ions are magnesium ions, calcium ions, barium ions, iron ions, copper ions, manganese ions, aluminum ions, zirconium ions, zinc ions, silver ions, gold ions, titanium ions, chromium ions, platinum ions, nickel ions, lead ions, arsenic ions, cadmium ions, mercury ions, antimony ions, or mixtures thereof.

Examples of more preferred metal ions are magnesium ions, calcium ions, iron ions, copper ions, aluminum ions, zirconium ions, zinc ions, silver ions, titanium ions, or mixtures thereof. Examples of even more preferred metal ions are magnesium ions, calcium ions, iron ions, copper ions, or mixtures thereof.

Examples of particularly preferred metal ions are iron ions or copper ions. Particularly preferred metal ions are Fe³⁺ ions or Cu²⁺ ions. Examples of also particularly preferred metal ions are magnesium ions or calcium ions. Also particularly preferred metal ions are Mg²⁺ ions or Ca²⁺ ions.

Metal ions can, for example, be brought into a cosmetic composition or a home care composition by using ingredients that contain metal ions as part of their structure or as impurities (e.g. natural impurities or catalyst residues). Examples of such ingredients are pigments, surfactants (which may contain, e.g., nickel catalysts), silicones (which may contain, e.g., platinum catalysts), clays, hydrocolloids, antiperspirant salts (e.g. aluminum salts, zirconium salts), organic salts (e.g. copper PCA, copper peptides), minerals (e.g. magnesium aluminum silicate, magnesium carbonate hydroxide absorbers, anticaking agents, slip modifiers), hair dyes, natural colors (e.g. henna, indigo), antiplaque actives (e.g. tin salts), soothing oral actives (e.g. strontium salts), sun care filters (e.g. zinc oxide, titanium dioxide).

According to the invention, a sugar amine (preferably N,N-dimethylglucamine) or a salt thereof is used in a cosmetic composition or a home care composition.

In one preferred embodiment, the sugar amine (preferably N,N-dimethylglucamine) or salt thereof is used in a cosmetic composition. In another preferred embodiment, the sugar amine (preferably N,N-dimethylglucamine) or salt thereof is used in a home care composition.

In preferred embodiments, the cosmetic composition is a skin care composition, hair care composition, scalp care composition or oral care composition. In preferred embodiments, the cosmetic composition is a skin care composition, hair care composition or scalp care composition. The cosmetic composition may, for example, be a rinse-off product or a leave-on product.

In preferred embodiments, the cosmetic composition is selected from liquid soaps, body washes, soap bars, creams, lotions, make up, wet wipes, deodorants, antiperspirants, shampoos, conditioners, coloring shampoos, hair dyes, and toothpaste. In preferred embodiments, the cosmetic composition is selected from liquid soaps, body washes, soap bars, creams, lotions, make up, wet wipes, deodorants, antiperspirants, shampoos, conditioners, coloring shampoos, and hair dyes.

Preferably, the sugar amine (preferably N,N-dimethylglucamine) or salt thereof is used in a cosmetic composition to increase the stability of the cosmetic composition, to provide or boost preservation, to prevent color degradation, to prevent fragrance degradation, to prevent rancidity, to prevent softening, to prevent brown-spotting, to prevent cracking, to prevent discoloration (e.g. discoloration due to metal ions or discoloration due to organic dyes), to enhance foaming, to enhance rinsability, to prevent off-odors, to prevent the degradation of active ingredients that may be present in the cosmetic composition, to increase the shelf life of the cosmetic composition, to increase the efficacy of vitamins, essential oils or fatty acids that may be present in the cosmetic composition, to provide antioxidant protection (e.g. when formulated with botanical active ingredients or vitamins), to prevent haze formation and precipitation, to boost antimicrobial agents, to preserve the stability of the cosmetic composition, to stabilize tint and color intensity (e.g. by stabilizing the redox system that may be present in the cosmetic composition), or to prevent the formation of reactive oxygen species (ROS) (e.g. ROS that are generated by iron or other metal catalysts upon mixing peroxide and base).

In a preferred embodiment, the cosmetic composition is selected from liquid soaps and body washes. Preferably, the sugar amine (preferably N,N-dimethylglucamine) or salt thereof is used in a cosmetic composition selected from liquid soaps and body washes to increase the stability of the cosmetic composition, to provide or boost preservation, to prevent color degradation, or to prevent fragrance degradation.

In a preferred embodiment, the cosmetic composition is a soap bar. Preferably, the sugar amine (preferably N,N-dimethylglucamine) or salt thereof is used in a cosmetic composition, which is a soap bar, to prevent rancidity, to prevent softening, to prevent brown-spotting, to prevent cracking, to prevent discoloration (e.g. discoloration due to metal ions), to enhance foaming, or to enhance rinsability.

In a preferred embodiment, the cosmetic composition is selected from creams, lotions, and make up. Preferably, the sugar amine (preferably N,N-dimethylglucamine) or salt thereof is used in a cosmetic composition selected from creams, lotions, and make up to prevent rancidity, to prevent discoloration (e.g. discoloration due to organic dyes), to prevent off-odors, to prevent the degradation of active ingredients that may be present in the cosmetic composition, to increase the shelf life of the cosmetic composition, to increase the efficacy of vitamins, essential oils or fatty acids that may be present in the cosmetic composition, or to boost preservation.

In a preferred embodiment, the cosmetic composition is make up. Preferably, the sugar amine (preferably N,N-dimethylglucamine) or salt thereof is used in a cosmetic composition, which is make up, to prevent rancidity, to increase the shelf life of the cosmetic composition, to increase the efficacy of vitamins, essential oils or fatty acids that may be present in the cosmetic composition, or to boost preservation. Also preferably, the sugar amine (preferably N,N-dimethylglucamine) or salt thereof is used in a cosmetic composition, which is make up, to stabilize colorants that may be present in the cosmetic composition, which may result in an increased color of the cosmetic composition.

In a preferred embodiment, the cosmetic composition is a wet wipe. Preferably, the sugar amine (preferably N,N-dimethylglucamine) or salt thereof is used in a cosmetic composition, which is a wet wipe, to prevent fragrance degradation, or to provide or boost preservation.

In a preferred embodiment, the cosmetic composition is selected from deodorants and antiperspirants. Preferably, the sugar amine (preferably N,N-dimethylglucamine) or salt thereof is used in a cosmetic composition selected from deodorants and antiperspirants to prevent fragrance degradation, or to provide antioxidant protection (e.g. when formulated with botanical active ingredients or vitamins).

In a preferred embodiment, the cosmetic composition is selected from shampoos and conditioners. Preferably, the sugar amine (preferably N,N-dimethylglucamine) or salt thereof is used in a cosmetic composition selected from shampoos and conditioners to prevent fragrance degradation, to prevent color degradation, to prevent haze formation and precipitation, or to boost antimicrobial agents.

In a preferred embodiment, the cosmetic composition is a coloring shampoo. Preferably, the sugar amine (preferably N,N-dimethylglucamine) or salt thereof is used in a cosmetic composition, which is a coloring shampoo, to preserve the stability of the cosmetic composition, or to stabilize tint and color intensity (e.g. by stabilizing the redox system that may be present in the cosmetic composition).

In a preferred embodiment, the cosmetic composition is a hair dye. Preferably, the sugar amine (preferably N,N-dimethylglucamine) or salt thereof is used in a cosmetic composition, which is a hair dye, to prevent the formation of reactive oxygen species (ROS) (e.g. ROS that are generated by iron or other metal catalysts upon mixing peroxide and base).

In a preferred embodiment, the cosmetic composition is a toothpaste. Preferably, the sugar amine (preferably N,N-dimethylglucamine) or salt thereof is used in a cosmetic composition, which is a toothpaste, to remove and prevent the buildup of bacterial plaque. Preferably, the sugar amine (preferably N,N-dimethylglucamine) or salt thereof is used in a cosmetic composition, which is a toothpaste, to remove and prevent the buildup of bacterial plaque while leaving healthy levels of calcium, achieving the chemical balance that is important for oral health.

In a preferred embodiment, the cosmetic composition is a hair care composition. Preferred hair care compositions are selected from shampoos, conditioners, coloring shampoos, and hair dyes.

The presence of heavy metals has detrimental effect on the hair when treating it with colorants, lighteners or various chemicals. Levels of metals deposited in the hair depend on the levels in tap water and on hair porosity value. Exposure to city pollution and washing hair with tap water makes metal particles to accumulate inside the hair fiber. The more porous the fiber, the greater the accumulation. Metals inside the fiber react with oxidant during hair coloration and bleaching. Metals in hair can also influence aesthetics of hair and scalp microbiome.

Sugar amines (such as Dimethylglucamine) are complexing agents, preferably chelating agents, with a strong affinity to metal ions and also by being alkaline agent opens up hair cuticle to bind better metal ions inside the hair fiber. When sugar amines (such as Dimethylglucamine) get in contact with metal ions, they prevent reaction with the oxidant. Another advantage is that sugar amines (such as Dimethylglucamine) are rather small molecules that penetrate easily, while conventional chelating agents are big multidentate molecules that have difficulty to penetrate hair fiber and therefore act only on surface of hair.

Preferably, the sugar amine (preferably N,N-dimethylglucamine) or salt thereof is used in a cosmetic composition, which is a hair care composition (preferably selected from shampoos, conditioners, coloring shampoos, and hair dyes), to prevent fragrance degradation, to prevent color degradation, to prevent haze formation and precipitation, to boost antimicrobial agents, to preserve the stability of the cosmetic composition, to stabilize tint and color intensity (e.g. by stabilizing the redox system that may be present in the cosmetic composition), or to prevent the formation of reactive oxygen species (ROS) (e.g. ROS that are generated by iron or other metal catalysts upon mixing peroxide and base).

In preferred embodiments, the home care composition is selected from automatic dishwashing products, hand dishwashing detergents, laundry detergents, fabric softeners, and surface cleaners. In preferred embodiments, the home care composition is selected from automatic dishwashing products, laundry detergents, fabric softeners, and surface cleaners.

Preferred automatic dishwashing products are automatic dishwashing rinse aids, automatic dishwashing detergents, automatic dishwashing machine cleaners, or automatic dishwashing auxiliaries. The automatic dishwashing products may, for example, be in the form of tablets, pouches, capsules, a powder, or a gel.

The laundry detergents may, for example, be in liquid form, or in the form of a powder, a compacted powder, a gel, a concentrate, pods, or in monodose form. The laundry detergent may, for example, be a laundry pretreatment product. The laundry detergents may, for example, be laundry detergents for color fabrics, white fabrics, delicate fabrics, fine fabrics, sportswear, or functional wear.

Preferred surface cleaners are kitchen cleaners, bathroom cleaners, toilet cleaners, or all purpose cleaners.

Preferably, the sugar amine (preferably N,N-dimethylglucamine) or salt thereof is used in a home care composition to remove stain, to remove grease, to increase the stability of the home care composition, to provide or boost preservation, to prevent color degradation, or to prevent fragrance degradation. Also preferably, the sugar amine (preferably N,N-dimethylglucamine) or salt thereof is used in a home care composition to meliorate the effect of hard water.

In a preferred embodiment, the sugar amine (preferably N,N-dimethylglucamine) or salt thereof is used in a home care composition to remove stain, or to remove grease.

In a preferred embodiment, the sugar amine (preferably N,N-dimethylglucamine) or salt thereof is used in a home care composition to increase the stability of the home care composition, to provide or boost preservation, to prevent color degradation, or to prevent fragrance degradation.

In a particular embodiment, the sugar amine (preferably N,N-dimethylglucamine) or salt thereof is used as a complexing agent, preferably as a chelating agent, and as a neutralizing agent.

In a particular embodiment, the sugar amine (preferably N,N-dimethylglucamine) or salt thereof is used as a complexing agent, preferably as a chelating agent, and as an alkaline agent.

Advantageously, sugar amine (preferably N,N-dimethylglucamine) or salts thereof can be used at a broad pH range.

In preferred embodiments, the sugar amine (preferably N,N-dimethylglucamine) or salt thereof is used as a complexing agent, preferably as a chelating agent, at a pH in the range of from 1 to 14, preferably from 3 to 12, more preferably from 4 to 11. Such pH ranges are particularly suitable for cosmetic applications or home care applications.

In preferred embodiments, the sugar amine (preferably N,N-dimethylglucamine) or salt thereof is used as a complexing agent, preferably as a chelating agent, at a pH in the range of from 4 to 10.5, preferably from 4 to 10, for example from 4 to 8, or from 9 to 10. Such pH ranges are particularly suitable for cosmetic applications.

In preferred embodiments, the sugar amine (preferably N,N-dimethylglucamine) or salt thereof is used as a complexing agent, preferably as a chelating agent, at a pH in the range of from 6 to 11, preferably from 8 to 10. Such pH ranges are particularly suitable for home care applications.

In preferred embodiments, the cosmetic composition is applied onto skin, hair or scalp. In preferred embodiments, the cosmetic composition is applied onto skin, hair or scalp and optionally removed from the skin, hair or scalp after application. In some embodiments, the cosmetic composition is applied onto skin, hair or scalp and removed from the skin, hair or scalp after application. In other embodiments, the cosmetic composition is applied onto skin, hair or scalp and not removed from the skin, hair or scalp after application. In another embodiment, the cosmetic composition, which is a toothpaste, is applied onto teeth.

In preferred embodiments, the home care composition is applied onto dishes, fabrics or surfaces. In preferred embodiments, the home care composition is applied onto dishes, fabrics or surfaces and typically removed from the dishes, fabrics or surfaces after application.

Advantageously, sugar amines (such as N,N-dimethylglucamine) or salts thereof can have multiple effects when used as a complexing agent, preferably as a chelating agent, in a cosmetic composition or a home care composition. Sugar amines (such as N,N-dimethylglucamine) or salts thereof can have a beneficial effect on the cosmetic composition or the home care composition and/or on a subject or a substrate onto which the composition is applied. For example, sugar amines (such as N,N-dimethylglucamine) or salts thereof can have a beneficial effect on the cosmetic composition and/or on a subject (for example on skin, hair or scalp) onto which the cosmetic composition is applied. For example, sugar amines (such as N,N-dimethylglucamine) or salts thereof can have a beneficial effect on the home care composition and/or on a substrate (for example on dishes, a fabric or a surface) onto which the home care composition is applied.

In one embodiment, the sugar amine (preferably N,N-dimethylglucamine) or a salt thereof is used as a replacement of ethylenediaminetetraacetic acid (EDTA) or a salt thereof.

The present invention also relates to the use of a sugar amine selected from N,N-dimethylglucamine, N-methylglucamine, N-methyl amino propane diol, N,N-dimethyl amino propane 1,2 diol, N-methylxylamine, N,N-dimethylxylamine, N-methylmaltosamine, N,N-dimethylmaltosamine, N-ethylglucamine, N,N-diethylglucamine, N-methylpropylglucamine, N-2-hydroxyethylglucamine, N,N-methyl 2-hydroxyethylglucamine, N-methylfructosamine, N,N-dimethylfructosamine, N-methylmaltulosamine, N,N-dimethylmaltulosamine, N-methylmaltotriosamine, N,N-dimethylmaltotriosamine, and mixtures thereof, or a salt thereof as a replacement of ethylenediaminetetraacetic acid (EDTA) or a salt thereof in a cosmetic composition or a home care composition. Preferred sugar amines are described further above. A particularly preferred sugar amine is N,N-dimethylglucamine.

The present invention also relates to the use of a sugar amine of the present invention (preferably N,N-dimethylglucamine) or a salt thereof to prepare a cosmetic composition or a home care composition.

In one aspect the sugar amine (preferably N,N-dimethylglucamine) or salt thereof is used to prepare a cosmetic composition. In another aspect the sugar amine (preferably N,N-dimethylglucamine) or salt thereof is used to prepare a home care composition.

The cosmetic composition, in which the sugar amines are used according to the appended claims, is a skin care composition, hair care composition, scalp care composition or oral care composition. In preferred embodiments, the cosmetic composition is a skin care composition, hair care composition or scalp care composition, or the cosmetic composition is selected from liquid soaps, body washes, soap bars, creams, lotions, make up, wet wipes, deodorants, antiperspirants, shampoos, conditioners, coloring shampoos, hair dyes, and toothpaste.

A home care composition, which the sugar amines are used as claimed, is selected from automatic dishwashing products, hand dishwashing detergents, laundry detergents, fabric softeners, and surface cleaners.

In preferred embodiments, respective cosmetic composition or home care composition comprises from 0.01 to 20 wt-%, preferably from 0.02 to 20 wt-%, more preferably from 0.1 to 15 wt-%, more preferably from 0.1 to 10 wt-%, even more preferably from 0.1 to 5 wt-%, even more preferably from 0.1 to 2 wt-%, particularly preferably from 0.1 to 1 wt-% of the sugar amine (wherein the sugar amine preferably is N,N-dimethylglucamine) or a salt thereof, based on the total weight of the cosmetic composition or home care composition.

The respective cosmetic compositions or home care compositions described herein can be prepared by methods known in the art. For example, the cosmetic composition or home care composition can be prepared by mixing the sugar amine (preferably N,N-dimethylglucamine) or a salt thereof with the other ingredients of the cosmetic composition or home care composition.

In at least one embodiment, the respective composition is a cosmetic composition. In at least one embodiment, the cosmetic composition is selected from the group consisting of shampoo, body wash, facial cleanser, cleansing masks, bubble bath, intimate wash, bath oil, cleansing milk, micellar water, make-up remover, cleansing wipes, perfume, liquid soaps, shaving soaps, shaving foams, cleansing foams, day creams, night creams, anti-ageing creams, body milks, body lotions, face serums, eye creams, sunscreen sprays, sun care milks, sun care creams, sun care gels, after-shave lotions, pre-shaving creams, depilatory creams, whitening creams, self-tanning creams, anti-acne gels, mascaras, foundations, primers, concealers, bb creams, eyeliners, highlighters, lip stains, hand sanitizers, nail varnish removers, conditioners, hair styling gels, hair styling creams, hair shine serums, scalp treatments, hair colourants, split end fluids, deodorants, antiperspirants, baby creams, insect repellent sprays, hand creams, foot creams, exfoliators, scrubs, and cellulite treatments.

In at least one embodiment, the composition comprises a carrier. In at least one embodiment, the carrier is a cosmetically acceptable carrier. In at least one embodiment, the composition comprises at least 10 wt.-% water. Water is useful for economic reasons but also because it is highly cosmetically acceptable. Optionally the composition comprises water-miscible or water-soluble solvents such as lower alkyl alcohols. In at least one embodiment, the composition comprises C₁-C₅ alkyl monohydric alcohols, preferably C₂-C₃ alkyl alcohols. The alcohols which may be present are in particular lower monohydric or polyhydric alcohols having 1 to 4 carbon atoms customarily used for cosmetic purposes, such as preferably ethanol and isopropanol.

In at least one embodiment, the composition comprises a water-soluble polyhydric alcohol. In at least one embodiment, the water-soluble polyhydric alcohols are polyhydric alcohols having two or more hydroxyl groups in the molecule. In at least one embodiment, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentanerythritol ether, and mixtures thereof.

In a preferred embodiment, the composition comprises a cosmetically acceptable carrier selected from the group consisting of water, glycols, ethanol, and combinations thereof.

In a preferred embodiment, the composition comprises an aqueous, alcoholic or aqueous-alcoholic carrier, and wherein the aqueous, alcoholic or aqueous-alcoholic carrier comprises water, ethanol, propanol, isopropanol, 1,2-propylene glycol, 1,3-propylene glycol, isobutanol, butanol, butyl glycol, butyl diglycol, glycerol, or a mixture thereof; preferably wherein the aqueous, alcoholic or aqueous-alcoholic solvent comprises water, ethanol, propanol, isopropanol, 1,2-propylene glycol, 1,3-propylene glycol, glycerol, or mixtures thereof; more preferably wherein the aqueous, alcoholic or aqueous-alcoholic carrier comprises water, isopropanol, 1,2-propylene glycol, 1,3-propylene glycol, or mixtures thereof; even more preferably wherein the aqueous, alcoholic or aqueous-alcoholic carrier consists of water or consists of a mixture of water and an alcohol wherein the alcohol is selected from the group consisting of isopropanol, 1,2-propylene glycol and 1,3-propylene glycol.

Natural carriers can also be used. In at least one embodiment, the composition comprises a carrier selected from the group consisting of plant oil, honey, plant-derived sugar compositions, and mixtures thereof.

In at least one embodiment, the composition has a viscosity of from 0 mPas to 200,000 mPas, or from 1 mPas to 200,000 mPas, or from 50,000 mPas to 200,000 mPas, or from 50,000 mPas to 100,000 mPas. In at least one embodiment, the composition has a viscosity of from 0.1 mPas to 50,000 mPas, or from 1 mPas to 20,000 mPas, or from 1 mPas to 10,000 mPas, or from 1 mPas to 5,000 mPas, or from 5 mPas to 3,500 mPas. The viscosity measurement conditions are defined in the definitions section above. Viscosity may be important for anti-drip reasons. Dripping can be inconvenient for the user. Furthermore, more viscous compositions can be useful for measured dispensing. In at least one embodiment, the composition has a viscosity of from 0 mPas to 1,000 mPas, or from 1 mPas to 1,000 mPas. This viscosity range is advantageous when the composition is in the form of a facial cleanser in view of the need for distribution on skin and ability to rinse off.

In at least one embodiment, the composition further comprises a viscosity-modifying substance. The viscosity-modifying substance is preferably a thickening polymer. In at least one embodiment, the thickening polymer selected from the group consisting of: copolymers of at least one first monomer type, which is chosen from acrylic acid and methacrylic acid, and at least one second monomer type, which is chosen from esters of acrylic acid and ethoxylated fatty alcohol; crosslinked polyacrylic acid; crosslinked copolymers of at least one first monomer type, which is chosen from acrylic acid and methacrylic acid, and at least one second monomer type, which is chosen from esters of acrylic acid with C₁₀- to C₃₀-alcohols; copolymers of at least one first monomer type, which is chosen from acrylic acid and methacrylic acid, and at least one second monomer type, which is chosen from esters of itaconic acid and ethoxylated fatty alcohol; copolymers of at least one first monomer type, which is chosen from acrylic acid and methacrylic acid, at least one second monomer type, which is chosen from esters of itaconic acid and ethoxylated C₁₀- to C₃₀-alcohol and a third monomer type, chosen from C₁-to C₄-aminoalkyl acrylates; copolymers of two or more monomers chosen from acrylic acid, methacrylic acid, acrylic esters and methacrylic esters; homopolymers of acryloyldimethyltaurate; crosslinked homopolymers of acryloyldimethyltaurate; copolymers of vinylpyrrolidone and salts of acryloyldimethyltaurate; copolymers of salts of acryloyldimethyltaurate and monomers chosen from esters of methacrylic acid and ethoxylated fatty alcohols; copolymers of salts of acryloyldimethyltaurate, methacrylic acid and monomers chosen from and alkylated acrylamid; salts of acryloyldimethyltaurate and N-vinylpyrrolidone; salts of acryloyldimethyltaurate, methacrylic acid and monomers chosen from esters of methacrylic acid; hydroxyethylcellulose; hydroxypropylcellulose; hydroxypropylguar; glyceryl polyacrylate; glyceryl polymethacrylate; copolymers of at least one C₂-, C₃- or C₄-alkylene and styrene; polyurethanes; hydroxypropyl starch phosphate; polyacrylamide; copolymer of maleic anhydride and methyl vinyl ether crosslinked with decadiene; carob seed flour; guar gum; xanthan; tara gum; dehydroxanthan; carrageenan; karaya gum; hydrolyzed corn starch; copolymers of polyethylene oxide, fatty alcohols and saturated methylenediphenyl diisocyanate (e.g. PEG-150/stearyl alcohol/SMDI copolymer); and mixtures thereof.

In at least one embodiment, the composition has a pH value of from 2.0 to 12.0, preferably from 3.0 to 9.0, more preferably from 4.5 to 8. By varying the pH value, a composition can be made available that is suitable for different applications.

In at least one embodiment, the composition comprises an alkalizing agent or pH adjusting agent. In at least one embodiment, ammonia or caustic soda is suitable, but water-soluble, physiologically tolerable salts of organic and inorganic bases can also be considered. In at least one embodiment, the pH adjusting agent is selected from ammonium hydrogen carbonate, ammonia, monoethanolamine, ammonium carbonate. In at least one embodiment, the alkalizing agents is selected from the group consisting of 2-amino-2-methyl-1-propanol, 2-amino-2-methyl-1,3-propanediol, 2-amino-2-ethyl-1,3-propanediol, tris(hydroxyl-methyl)-aminomethane, 2-amino-1-butanole, tris-(2-hydroxypropyl)-amine, 2,2-iminobisethanol, lysine, iminourea (guanidine carbonate), tetrahydro-1,4-oxazine, 2-amino-5-guanidin-valeric acid, 2-aminoethansulfonic acid, diethanolamine, triethanolamine, N-methyl ethanolamine, isopropanolamine, diisopropanolamine, triisopropanolamine, glucamine, sodium hydroxide, potassium hydroxide, lithium hydroxide and magnesium oxide, and mixtures thereof.

To establish an acidic pH value, and acid can be included. In at least one embodiment, the composition comprises an acid selected from the group consisting of hydrochloric acid, phosphoric acid, acetic acid, formic acid, sulfuric acid, hydrochloric acid, citric acid, and mixtures thereof. Citric acid is most preferred in that it has high consumer acceptance. In at least one embodiment, the acidic pH is adjusted with a buffer such as a phosphate buffer, a TRIS buffer or a citric buffer. The buffers may be used alone or in combination with an acid.

In at least one embodiment, the composition is in liquid form. In an alternative embodiment, the composition is in solid form. Optionally, the composition is in powdered or granulated form. This is advantageous in that it is not needed to ship liquid, which is typically heavy over long distances, and thus has economic and environmental benefits. A solid form can be achieved by spray drying the composition, the employment of a rotary evaporator, by drying on trays at temperature above the boiling point of the solvent, in vacuum, by drying in the reactor, in vertical or horizoantal powder dryers, by atmospheric, pressure or vacuum filtration with subsequent drying of the wet filtrate. The composition can be converted into liquid form after it has been shipped e.g. by adding water.

In at least one embodiment, the composition comprises a surfactant or surfactant system. In at least one embodiment, the surfactant or surfactant system comprises a surfactant selected from the group consisting of anionic surfactants, cationic surfactants, non-ionic surfactants, zwitterionic surfactants and/or amphoteric surfactants.

In at least one embodiment, the composition comprises a total amount of surfactant of from 0.01 wt.-% to 70 wt.-%, from 0.1 wt.-% to 40%, from 1 wt.-% to 30%, from 2 wt.-% to 20 wt.-%.

In at least one embodiment, the composition comprises an anionic surfactant. In at least one embodiment, the anionic surfactant is selected from the group consisting of (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, α-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono-, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof.

In at least one embodiment, the composition comprises an acylglycinate surfactant. In at least one embodiment, the acylglycinate surfactant conforms to the formula (Y): 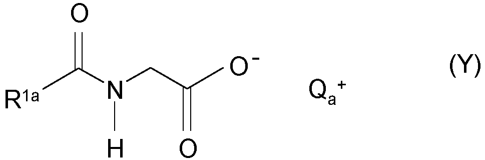 wherein
- R^{1a}: is a linear or branched, saturated alkanoyl group having 6 to 30, preferably 8 to 22, particularly preferably 8 to 18, carbon atoms or is a linear or branched, mono- or polyunsaturated alkenoyl group having 6 to 30, preferably 8 to 22 and particularly preferably 12 to 18 carbon atoms, and
- Qₐ⁺: is a cation. In at least one embodiment, Qₐ⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. Optionally R^{1a} is, independently from one another, selected from (C₁-C₂₂)-alkyl radicals or (C₂-C₁₀)-hydroxyalkyl radicals. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C₁₂ alkyl or C₁₄ alkyl. In at least one embodiment, the acylglycinate surfactant is selected from those conforming to formula (Y), wherein R is C₁₆ alkyl or C₁₈ alkyl.

In at least one embodiment, the composition comprises a glutamate surfactant corresponding to formula (Z) or a salt thereof: 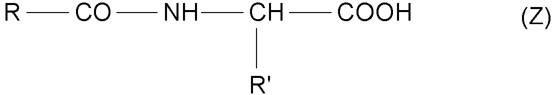 wherein
- R': is HOOC-CH₂-CH₂- or M⁺⁻OOC-CH₂-CH₂- wherein M⁺ is a cation; and wherein R is a linear or branched, saturated alkanoyl group having 6 to 30, preferably 8 to 22, more preferably 8 to 18, carbon atoms or is a linear or branched, mono- or polyunsaturated alkenoyl group having 6 to 30, preferably 8 to 22 and more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the glutamate surfactant is selected from sodium cocoyl glutamate and potassium cocoyl glutamate. In at least one embodiment, the glutamate surfactant is selected from those conforming to formula (Z), wherein R is C₁₂ alkyl or C₁₄ alkyl. In at least one embodiment, the glutamate surfactant is selected from those conforming to formula (Z), wherein R is C₁₆ alkyl or C₁₈ alkyl.

In at least one embodiment, the composition comprises from 0.01 wt.-% to 30 wt.-%, or 1 wt.-% to 25 wt.-%, preferably from 5 wt.-% to 20 wt.-%, more preferably from 12 wt.-% to 18 wt.-% anionic surfactant.

In at least one embodiment, the composition comprises a non-ionic surfactant.

In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain comprising from 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of from 2 to 30 mol of ethylene oxide and/or 1 to 5 mol of propylene oxide onto C₈- to C₂₂-fatty alcohols, onto C₁₂- to C₂₂-fatty acids or onto alkyl phenols having 8 to 15 carbon atoms in the alkyl group, C₁₂- to C₂₂-fatty acid mono- and diesters of addition products of from 1 to 30 mol of ethylene oxide onto glycerol, addition products of from 5 to 60 mol of ethylene oxide onto castor oil or onto hydrogenated castor oil, fatty acid sugar esters, in particular esters of sucrose and one or two C₈- to C₂₂-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C₈- to C₂₂-fatty acids and a degree of ethoxylation of from 4 to 20, polyglyceryl fatty acid esters, in particular of one, two or more C₈- to C₂₂-fatty acids and polyglycerol having preferably 2 to 20 glyceryl units, alkyl glucosides, alkyl oligoglucosides and alkyl polyglucosides having C₈ to C₂₂-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (Pluronics^{®}), fatty acid alkylol amides, (fatty acid amide polyethylene glycols), N-alkyl-, N-alkoxypolyhydroxy-fatty acid amide, sucrose esters, sorbitol esters, polyglycol ethers, and mixtures thereof.

In at least one embodiment, the composition comprises a fatty N-methyl-N-glucamide surfactant. In at least one embodiment, the fatty N-methyl-N-glucamide surfactant conforms to the formula (X): 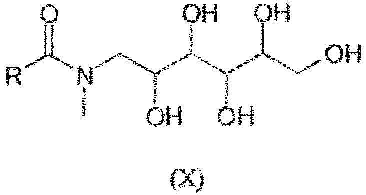 wherein
- R: is a linear or branched alkyl or alkenyl group having from 3 to 30 carbon atoms. In at least one embodiment, R is an alkyl group having from 3 to 30 carbon atoms.

In at least one embodiment, R is a saturated aliphatic hydrocarbon group which can be linear or branched and can have from 3 to 20 carbon atoms in the hydrocarbon chain, preferably linear or branched. Branched means that a lower alkyl group such as methyl, ethyl or propyl is present as substituent on a linear alkyl chain. In at least one embodiment, R is selected from the group consisting of 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-nonyl, 1-decyl, 1-undecyl, 1-dodecyl, 1-tetradecyl, 1-hexadecyl and 1-octadecyl. Suitable fatty N-methyl-N-glucamide surfactants are described in WO2013/178700 and EP0550637, which are incorporated herein by reference. In at least one embodiment, the N-methyl-N-glucamide surfactant is selected from those conforming to formula (X), wherein R is C₁₂ alkyl or C₁₄ alkyl. In at least one embodiment, the N-methyl-N-glucamide surfactant is selected from those conforming to formula (X), wherein R is C₁₆ alkyl or C₁₈ alkyl. N-methyl-N-glucamide surfactants are available from Clariant under their GlucoTain^{®} brand.

In at least one embodiment, the composition comprises from 1 wt.-% to 20 wt.-%, more preferably from 2 wt.-% to 10 wt.-%, even more preferably from 3 wt.-% to 7 wt.-% non-ionic surfactant.

In at least one embodiment, the composition comprises capryloyl/caproyl anhydro methyl glucamide (Velsan Flex).

In at least one embodiment, the amphoteric surfactants are selected from the group consisting of N-(C₁₂-C₁₈)-alkyl-β-aminopropionates and N-(C₁₂-C₁₈)-alkyl-β-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine, (C₁₂-C₁₈)-alkyl-dimethylsulfopropylbetaine, amphosurfactants based on imidazoline (trade name: Miranol^{®}, Steinapon^{®}), preferably the sodium salt of 1-(β-carboxymethyloxyethyl)-1-(carboxymethyl)-2-laurylimidazolinium; amine oxide, e.g., (C₁₂-C₁₈)-alkyl-dimethylamine oxide, fatty acid amidoalkyldimethylamine oxide, and mixtures thereof.

In at least one embodiment, the composition comprises a betaine surfactant. Optionally, the betaine surfactant is selected from C₈- to C₁₈-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. Optionally, the betaine surfactant is selected from C₈- to C₁₈-sulfobetaines. In at least one embodiment, the betaine surfactant is selected from the group consisting of cocodimethylsulfopropylbetaine, stearyldimethylsulfopropylbetaine, lauryldimethyl-sulfoethylbetaine, laurylbis(2-hydroxyethyl)sulfopropylbetaine, and combinations thereof. Optionally, the betaine surfactant is selected from carboxyl derivatives of imidazole, the C₈- to C₁₈-alkyldimethylammonium acetates, the C₈- to C₁₈-alkyldimethylcarbonylmethylammonium salts, and the C₈- to C₁₈-fatty acid alkylamidobetaines, and mixtures thereof. Optionally, the C₈- to C₁₈-fatty acid alkylamidobetaine is selected from coconut fatty acid amidopropylbetaine, N-coconut fatty acid amidoethyl-N-[2-(carboxymethoxy)ethyl]glycerol (CTFA name: Cocoamphocarboxyglycinate), and mixtures thereof.

In at least one embodiment, the composition comprises from 0.5 wt.-% to 20 wt.-%, preferably from 1 wt.-% to 10 wt.-% amphoteric surfactant.

In at least one embodiment, the composition comprises a surfactant system. In at least one embodiment, the surfactant system comprises at least one surfactant selected from the group consisting of lauryl sulfate, laureth sulfate, cocoamido-propylbetaine, sodium cocoylglutamate, lauroamphoacetate, and mixtures thereof. In at least one embodiment, the surfactant system comprises sodium laureth sulphate, sodium lauryl sulphate, and optionally cocamidopropyl betaine. In at least one embodiment, the surfactant system comprises sodium laureth sulphate, Potassium Cocyl Glutamate, and cocamidopropyl betaine.

In at least one embodiment, the composition comprises a conditioning agent. In at least one embodiment, the conditioning agent is a water insoluble, water dispersible, non-volatile, liquid that forms emulsified, liquid particles. In at least one embodiment, the conditioning agent is a silicone (e.g., silicone oil, cationic silicone, silicone gum, high refractive silicone, and silicone resin), an organic conditioning oil (e.g., hydrocarbon oils, polyolefins, and fatty esters), or combinations thereof. In at least one embodiment, the composition comprises lauryl/myristyl polyricinoleate (Genadvance Hydra).

In at least one embodiment, the conditioning agent is a silicone, and wherein the composition comprises from 0.01 wt.-% to 10 wt.-%, or from 0.1 wt.-% to 5 wt.-% silicone conditioning agent, by total weight of the composition. Suitable silicone conditioning agents, and optional suspending agents for the silicone, are described in US 5,104,646. In at least one embodiment, the composition comprises a silicone gum selected from the group consisting of polydimethylsiloxane, (polydimethylsiloxane) (methylvinylsiloxane) copolymer, poly(dimethylsiloxane) (diphenylsiloxane) (methylvinylsiloxane) copolymer, and mixtures thereof.

In at least one embodiment, the composition comprises a terminal aminosilicone. "Terminal aminosilicone" as defined herein means silicone comprising one or more amino groups at one or both ends of the silicone backbone. In at least one embodiment, the composition is substantially free of any silicone compound comprising pendant amino groups. In an embodiment, the composition is substantially free of any silicone compound other than terminal aminosilicones. In at least one embodiment, the amino group at least one terminus of the silicone backbone of the terminal aminosilicone is selected from the group consisting of primary amines, secondary amines and tertiary amines. In at least one embodiment, the composition comprises a terminal aminosilicone conforming to Formula (S):

(R^{F})ₐG₃₋ₐ-Si-(-OSiG₂)ₙ-O-SiG₃₋ₐ(R^{F})ₐ (S)

wherein
- G: is hydrogen, phenyl, hydroxy, or C₁-C₈ alkyl, preferably methyl;
- a: is an integer having a value from 1 to 3, preferably 1;
- b: is 0, 1 or 2, preferably 1;
- n: is a number from 0 to 1,999;
- R^{F}: is a monovalent radical conforming to the general formula C_{q}H_{2q}L, wherein q is an integer having a value from 2 to 8 and
- L: is selected from the following groups: -N(R^{T})CH₂-CH₂-N(R^{T})₂; -N(R^{T})₂; -N(R^{T})₃A⁻; -N(R^{T})CH₂-CH₂-NR^{T}H₂A⁻; wherein R^{T} is hydrogen, phenyl, benzyl, or a saturated hydrocarbon radical, preferably an alkyl radical from having from 1 to 20 carbon atoms; A⁻ is a halide ion.

In at least one embodiment, the terminal aminosilicone corresponding to Formula (S) has a=1, q=3, G=methyl, n is from 1000 to 2500, alternatively from 1500 to 1700; and L is -N(CH₃)₂. A suitable terminal aminosilicone corresponding to Formula (S) has a=O, G=methyl, n is from 100 to 1500, or from 200 to 800, and L is selected from the following groups: -N(R^{T})CH₂-CH₂-N(R^{T})₂; -N(R^{T})₂; -N(R^{T})₃A⁻; -N(R^{T})CH₂-CH₂-NR^{T}H₂A⁻; wherein R^{T} is selected from hydrogen, phenyl, benzyl, or a saturated hydrocarbon radical, preferably an alkyl radical from having from 1 to 20 carbon atoms; A⁻ is a halide ion, alternatively L is -NH₂. In at least one embodiment, the terminal aminosilicone is selected from the group consisting of bis-aminomethyl dimethicone, bisaminoethyl dimethicone, bis-aminopropyl dimethicone, bis-aminobutyl dimethicone, and mixtures thereof. In at least one embodiment, the viscosity of the terminal aminosilicone is from 1,000 to 30,000 mPas, or from 5,000 to 20,000 mPas measured at 25 °C.

In at least one embodiment, the composition comprises from 0.1 wt.-% to 20 wt.-%, or from 0.5 wt.-% to 10 wt.-%, or from 1 wt.-% to 6 wt.-% terminal aminosilicone, by total weight of the composition.

In at least one embodiment, the composition comprises a high melting point fatty compound. The high melting point fatty compound has a melting point of 25 °C or higher. In at least one embodiment, the high melting point fatty compound is selected from the group consisting of a fatty alcohol, fatty acid, fatty alcohol derivative, fatty acid derivative, and mixtures thereof. Non-limiting examples of the high melting point compounds are found in International Cosmetic Ingredient Dictionary, Fifth Edition, 1993, and CTFA Cosmetic Ingredient Handbook, Second Edition, 1992. The composition may comprise from 0.1 wt.-% to 40 wt.-%, or from 1 wt.-% to 30 wt.-%, or from 1.5 wt.-% to 16 wt.-%, or from 1.5 wt.-% to 8 wt.-% of a high melting point fatty compound, by total weight of the composition. This is advantageous in view of providing improved conditioning benefits such as slippery feel during the application to wet hair, softness and moisturized feel on dry hair. In at least one embodiment, the fatty alcohol is selected from the group consisting of: cetyl alcohol, stearyl alcohol, behenyl alcohol, and mixtures thereof. In at least one embodiment, the composition comprises a linear fatty alcohol, wherein the linear fatty alcohol is also comprised in a lamellar gel matrix. The lamellar gel matrix is suitable for providing various conditioning benefits such as slippery feel during the application to wet hair and softness and moisturized feel on dry hair. The linear fatty alcohol may comprise from 8 to 24 carbon atoms. In at least one embodiment, the linear fatty alcohol is selected from the group consisting of: cetyl alcohol, stearyl alcohol, and mixtures thereof. In an embodiment, the weight ratio of total linear fatty alcohol to terminal aminosilicone is from 0.5:1 to 10:1, or from 1:1 to 5:1, or from 2.4:1 to 2.7:1. In at least one embodiment, the lamellar gel matrix comprises a cationic surfactant and a high melting point fatty compound. In view of providing the lamellar gel matrix, the cationic surfactant and the high melting point fatty compound are contained at a level such that the weight ratio of the cationic surfactant to the high melting point fatty compound is in the range of from 1: 1 to 1: 10, or from 1: 1 to 1:6.

In at least one embodiment, the composition comprises a cationic surfactant. In at least one embodiment, the composition comprises from 0.05 wt.-% to 3.0 wt.-%, or from 0.075 wt.-% to 2.0 wt.-%, or from 0.1 wt.-% to 1.0 wt.-%, of cationic surfactant by total weight of the composition. In at least one embodiment, the cationic surfactant is comprised in a lamellar gel matrix. In other words, the composition comprises a lamellar gel matrix and the lamellar gel matrix comprises the cationic surfactant. In an embodiment, cationic surfactant is according to Formula (C): 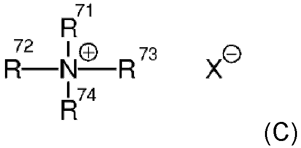 wherein
at least one of R⁷¹, R⁷², R⁷³ and R⁷⁴ is selected from an aliphatic group of from 8 to 30 carbon atoms, an aromatic, alkoxy, polyoxyalkylene, alkylamido, hydroxyalkyl, aryl, or an alkylaryl group having up to 22 carbon atoms;
the remainder of R⁷¹, R⁷², R⁷³ and R⁷⁴ are independently selected from the group consisting of an aliphatic group consisting of from 1 to 22 carbon atoms, and an aromatic, alkoxy, polyoxyalkylene, alkylamido, hydroxyalkyl, aryl or alkylaryl group having up to 22 carbon atoms;
X is selected from the group consisting of: halogen, acetate, citrate, lactate, glycolate, phosphate, nitrate, sulfonate, sulfate, alkylsulfate, alkyl sulfonate radicals, and combinations thereof.

In at least one embodiment, the cationic surfactant is selected from the group consisting of behenyl trimethyl ammonium chloride, methyl sulfate or ethyl sulfate, and stearyl trimethyl ammonium chloride, methyl sulfate or ethyl sulfate. It is believed that a longer alkyl group provides improved smoothness and soft feeling on wet and dry hair, compared to cationic surfactants with a shorter alkyl group. It is also believed that such cationic surfactants can provide reduced irritation, compared to those having a shorter alkyl group.

In at least one embodiment, the cationic surfactant is a di-long alkyl quatemized ammonium salt selected from the group consisting of: dialkyl (14 - 18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof.

In at least one embodiment, the cationic surfactant is a tertiary amido amine having an alkyl group of from 12 to 22 carbons. The tertiary amido amine may be selected from the group consisting of stearamidopropyldimethyl-, stearamidopropyldiethyl-, stearamidoethyldiethyl-, stearamidoethyldimethyl-, palmitamidopropyldimethyl-, palmitamidopropyldiethyl-, palmitamidoethyldiethyl-, palmitamidoethyldimethyl-, behenamidopropyldimethyl-, behenamidopropyldiethyl-, behenamidoethyldiethyl-, behenamidoethyldimethyl-, arachidamidopropyldimethyl-, arachidamidopropyldiethyl-, arachidamidoethyldiethyl-, and arachidamidoethyldimethyl-amine, diethylaminoethylstearamide, and mixtures thereof. A tertiary amido amine may be used in combination with an acid. The acid is typically used as a salt-forming anion. In an embodiment, the acid is selected from the group consisting of lactic acid, malic acid, hydrochloric acid, 1-glumatic acid, acetic acid, citric acid, and mixtures thereof.

In at least one embodiment, the cationic surfactant is selected from the group consisting of cetyltrimonium chloride (CTAC), stearyltrimonium chloride (STAC), behentrimonium methosulfate, stearoylamidopropyldimethyl amine (SAPDMA), distearyldimethylammonium chloride, and mixtures thereof.

In at least one embodiment, the composition comprises additives common in cosmetology, pharmacy, and dermatology, which are hereinafter called auxiliaries. In at least one embodiment, the auxiliary is selected from the group consisting of oily substances, emulsifiers, coemulsifiers, cationic polymers, film formers, superfatting agents, stabilizers, active biogenic substances, glycerol, preservatives, pearlizing agents, dyes and fragrances, solvents, opacifiers, functional acids, and also protein derivatives such as gelatin, collagen hydrolysates, natural or synthetic-based polypeptides, egg yolk, lecithin, lanolin and lanolin derivatives, fatty alcohols, silicones, deodorants, substances with a keratolytic and keratoplastic action, enzymes, and/or carriers/solvents.

In at least one embodiment, the composition comprises water soluble vitamins and their derivatives, water soluble amino acids and their salts and/or derivatives, viscosity modifiers, dyes, nonvolatile solvents or diluents (water soluble and insoluble), pearlescent aids, thickeners, foam boosters, additional surfactants or nonionic co-surfactants, pediculocides, pH adjusting agents, perfumes, preservatives, chelants, proteins, skin active agents, sunscreens, UV absorbers, vitamins, niacinamide, caffeine, minoxidil, and combinations thereof. In at least one embodiment, the composition comprises from 0 wt.-% to 5 wt.-% vitamins and amino acids, by total weight of the composition. The composition may also comprise pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C.I. Names. The composition may comprise from 0 wt.-% to 5 wt.-% pigment materials. The composition may comprise from 0 wt.-% to 5 wt.-% antimicrobial agents.

In at least one embodiment, the composition comprises an oily substance, which is any fatty substance which is liquid at room temperature (25 °C). In at least one embodiment, the composition comprises oily substance selected from the group consisting of silicone oils, volatile or nonvolatile, linear, branched or cyclic, optionally with organic modification; phenylsilicones; silicone resins and silicone gums; mineral oils such as paraffin oil or vaseline oil; oils of animal origin such as perhydrosqualene, lanolin; oils of plant origin such as liquid triglycerides, e.g., sunflower oil, corn oil, soybean oil, rice oil, jojoba oil, babusscu oil, pumpkin oil, grapeseed oil, sesame oil, walnut oil, apricot oil, macadamia oil, avocado oil, sweet almond oil, lady's-smock oil, castor oil, triglycerides of caprylic/capric acids, olive oil, peanut oil, rapeseed oil, argan oil, abyssinian oil, and coconut oil; synthetic oils such as purcellin oil, isoparaffins, linear and/or branched fatty alcohols and fatty acid esters, preferably guerbet alcohols having 6 to 18, preferably 8 to 10, carbon atoms; esters of linear (C₆-C₁₃) fatty acids with linear (C₆-C₂₀) fatty alcohols; esters of branched (C₆-C₁₃) carboxylic acids with linear (C₆-C₂₀) fatty alcohols, esters of linear (C₆-C₁₈) fatty acids with branched alcohols, especially 2-ethylhexanol; esters of linear and/or branched fatty acids with polyhydric alcohols (such as dimerdiol or trimerdiol, for example) and/or guerbet alcohols; triglycerides based on (C₆-C₁₀) fatty acids; esters such as dioctyl adipate, diisopropyl dimer dilinoleate; propylene glycols/dicaprylate or waxes such as beeswax, paraffin wax or microwaxes, alone or in combination with hydrophilic waxes, such as cetylstearyl alcohol, for example; fluorinated and perfluorinated oils; fluorinated silicone oils; mixtures of the aforementioned compounds.

In at least one embodiment, the composition comprises a non-ionic coemulsifier. In at least one embodiment, the non-ionic coemulsifier is selected from adducts of from 0 to 30 mol of ethylene oxide and/or from 0 to 5 mol of propylene oxide with linear fatty alcohols having 8 to 22 carbon atoms, with fatty acids having 12 to 22 carbon atoms, with alkylphenols having 8 to 15 carbon atoms in the alkyl group, and with sorbitan or sorbitol esters; (C₁₂-C₁₈) fatty acid monoesters and diesters of adducts of from 0 to 30 mol of ethylene oxide with glycerol; glycerol monoesters and diesters and sorbitan monoesters and diesters of saturated and unsaturated fatty acids having 6 to 22 carbon atoms and, where appropriate, their ethylene oxide adducts; adducts of from 15 to 60 mol of ethylene oxide with castor oil and/or hydrogenated castor oil; polyol esters and especially polyglycerol esters, such as polyglyceryl polyricinoleate and polyglyceryl poly-12-hydroxystearate, for example. Likewise suitable are mixtures of compounds from one or more of these classes of substance. Examples of suitable ionogenic coemulsifiers include anionic emulsifiers, such as mono -, di- or tri-phosphoric esters, but also cationic emulsifiers such as mono-, di-, and tri-alkyl quats and their polymeric derivatives.

In at least one embodiment, the composition comprises a cationic polymer. Suitable cationic polymers include those known under the INCI designation "Polyquaternium", especially Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, and also Polyquaternium 37 & mineral oil & PPG trideceth (Salcare SC95), PVP-dimethylaminoethyl methacrylate copolymer, guar-hydroxypropyltriammonium chlorides, and also calcium alginate and ammonium alginate, and also Polyquaternium-116 (Genadvance Life). It is additionally possible to employ cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as amidomethicones, for example; copolymers of adipic acid and dimethylaminohydroxypropyldiethylenetriamine; polyaminopolyamide and cationic chitin derivatives, such as chitosan, for example.

In at least one embodiment, the composition comprises a superfatting agent. As superfatting agents it is possible to use substances such as, for example, polyethoxylated lanolin derivatives, lecithin derivatives, polyol fatty acid esters, monoglycerides, and fatty acid alkanol amides, the latter serving simultaneously as foam stabilizers. Moisturizers available include for example isopropyl palmitate, glycerol and/or sorbitol.

In at least one embodiment, the composition comprises a stabiliser. As stabiliser it is possible to use metal salts of fatty acids, such as magnesium, aluminum and/or zinc stearate, for example.

In at least one embodiment, the composition comprises a care additive. The compositions can be blended with conventional ceramides, pseudoceramides, fatty acid N-alkylpolyhydroxyalkyl amides, cholesterol, cholesterol fatty acid esters, fatty acids, triglycerides, cerebrosides, phospholipids, panthenol and similar substances as a care additive.

In at least one embodiment, the composition comprises a preservative or preservative system. Examples of suitable preservatives include benzyl alcohol, piroctone olamine, phenoxyethanol, parabens, pentanediol, benzoic acid/sodium benzoate, sorbic acid/potassium sorbate, and other organic acids used to provide antimicrobial protection. Preservation boosting ingredients include anisic acid, lactic acid, sorbitan caprylate, ethylhexylglycerin, caprylyl glycol, octanediol, and similar substances. In at least one embodiment, the composition comprises 0.01 to 5 wt.-%, particularly preferably from 0.05 wt.-% to 1 wt.-% of at least one preservative. Suitable preservatives are the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition comprises a preservative selected from the group consisting of cetyltrimethyl ammoniumchloride, cetylpyridinium chloride, benzethonium chloride, diisobutylethoxyethyldimethyl benzylammoniumchloride, sodium N-lauryl sarcosinate, sodium-N-palmethylsarcosinate, Lauroylsarcosine, N-myristoylglycine, potassium-N-laurylsarcosine, trimethylammoniumchloride, sodium aluminium chlorohydroxylactate, triethylcitrate, tricetylmethylammoniumchloride, 2,4,4'-trichloro-2'-hydroxydiphenylether (Triclosan), phenoxyethanol, 1,5-pentandiol, 1,6-hexandiol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, Octopirox^{®}, methylchloroisothiazolinone, methylisothiazolinone, methyldibromo glutaronitrile, AgCl, chloroxylenol, sodium salts of diethylhexylsulfosuccinate, sodiumbenzoate, phenoxyethanol, benzylalkohol, phenoxyisopropanol, paraben, such as butyl-, ethyl-, methyl- und propylparaben, and their salts, pentandiol, 1,2-octanediol, ethylhexylglycerin, benzylalcohol, caprylyl glyceryl ether (Velsan CGE), octylglycerin, sorbic acid, benzoic acid, lactic acid, anisic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition is substantially free of parabens.

In at least one embodiment, the composition comprises an anti-fungal substance. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, zinc pyrithione, octopirox, and combinations thereof. In at least one embodiment, the composition comprises a total amount of anti-fungal substance in the composition of from 0.1 wt.-% to 1 wt.-%. In at least one embodiment, the composition comprises a pyridinethione anti-dandruff particulates, for example 1-hydroxy-2-pyridinethione salts, are highly preferred particulate anti-dandruff agents. The concentration of pyridinethione antidandruff particulate may ranges from 0.1 % to 4 %, by weight of the composition, preferably from 0.1 % to 3 %, more preferably from 0.3 % to 2 %. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable. Pyridinethione anti-dandruff agents are described, for example, in US2809971; US3236733; US3753196; US3761418; US4345080; US4323683; US4379753; and US4470982. It is contemplated that when ZPT is used as the anti-dandruff particulate in the compositions herein, that the growth or regrowth of hair may be stimulated or regulated, or both, or that hair loss may be reduced or inhibited, or that hair may appear thicker or fuller.

Functional acids are acidic substances used to impart a clinical functionality to the skin or hair upon application. Suitable functional acids include alpha hydroxy acids, beta-hydroxy acids, lactic acid, retinoic acid, and similar substances.

In at least one embodiment, the composition comprises an astringent. In at least one embodiment, the astringent is selected from the group consisting of magnesium oxide, aluminium oxide, titanium dioxide, zirconium dioxide, zinc oxide, oxide hydrates, aluminium oxide hydrate (boehmite) and hydroxide, chlorohydrates of calcium, magnesium, aluminium, titanium, zirconium or zinc. In at least one embodiment, the composition comprises from 0.001 wt.-% to 10 wt.-%, or from 0.01 wt.-% to 9 wt.-%, or from 0.05 wt.-% to 8 wt.-%, or from 0.1 wt.-% to 5 wt.-% astringent.

In at least one embodiment, the composition comprises a deodorising agent. In at least one embodiment, the deodorising agent is selected from the group consisting of allantoin, bisabolol, and combinations thereof. In at least one embodiment, the deodorising agent comprises Magnesium Hydroxide, Magnesium Carbonate Hydroxide, and combinations thereof, preferably Magnesium Hydroxide and Magnesium Carbonate Hydroxide. Such a deodorizing agent is commercially available as Caremag^{™}. In at least one embodiment, the composition comprises from 0.001 wt.-% to 10 wt.-%, or from 0.01 wt.-% to 9 wt.-%, or from 0.05 wt.-% to 8 wt.-%, or from 0.1 wt.-% to 5 wt.-% deodorising agent.

In at least one embodiment, the composition comprises a sun protection agent and/or UV filter. Suitable sun protection agents and UV filters are disclosed in WO2013017262A1 (published on 7th Feb 2013), from page 32, line 11 to the end of page 33. In at least one embodiment, the sun protection agent and/or UV filter is selected from the group consisting of 4-amino benzoic acid, 3-(4'-trimethylammonium)-benzylide-boran-2-one-methylsulfate, camphor benzalkonium methosulfate, 3,3,5-trimethyl-cyclohexylsalicylate, 2-hydroxy-4-methoxybenzophenone, 2-phenylbenzimidazole-5-sulfonic acid and potassium-, sodium- und triethanolamine salts thereof, 3,3'-(1,4-phenylene dimethine)-bis-(7,7-dimethyl-2-oxobicyclo[2.2.1]-heptane-1-methane sulfonic acid) and its salts, 1-(4-tert.-butylphenyl)-3-(4-methoxyphenyl)propan-1,3-dion, 3-(4'-sulfo)-benzylidene-bornane-2-one its salts, 2-cyan-3,3-diphenyl-acrylic acid-(2-ethylhexylester), polymers of N-[2(and 4)-(2-oxoborn-3-ylidenmethyl)benzyl]-acrylamide, 4-methoxycinnamic acid-2-ethyl-hexylester, ethoxylated ethyl-4-amino-benzoate, 4-methoxycinnamic acid-isoamylester, 2,4,6-tris-[p-(2-ethylhexyloxycarbonyl)anilino]-1,3,5-triazine, 2-(2H-benzotriazole-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-aminocarbonyl)phenylamino]-1,3,5-triazin-2,4-yl)diimino]bis-(benzoic acid-2-ethylhexylester), 3-benzophenone, 4-benzophenone (acic), 3(4'-methylbenzyliden)-D,L-camphor, 3-benzylidene-camphor, salicylic acid-2-ethylhexylester, 4- dimethyl aminobenzic acid-2-ethylhexylester, hydroxy-4-methoxy-benzophenone-5 sulfonic acid and the sodium salt thereof, 4-isopropyl benzylsalicylate, N,N,N-trimethyl-4-(2-oxoborn-3-ylidenemethyl) anilium methyl sulfate, homosalate (INN), oxybenzone (INN), 2-phenylbenzimidazole-5-sulfonic acid and its sodium, potassium, and triethanolamine salts, octylmethoxy cinnamic acid, isopentyl-4-methoxy cinnamic acid, isoamyl-p-methoxy cinnamic acid, 2,4,6-trianilino-(p-carbo-2'-ethylhexyl-1'-oxy)-1,3,5-triazine (octyl triazone) phenol, 2,2(2H-benzotriazol-2-yl)-4-methyl-6-(2-methyl-3-(1,3,3,3-tetramethyl-1-(trimethylsilyl)oxy)-disiloxanyl)propyl (drometrizole trisiloxane) benzic acid, 4,4-((6-(((1,1-dimethylethyl)amino)carbonyl)phenyl)amino)-1,3,5-triazine-2,4-diyl)diimino)bis,bis(2-ethylhexyl)ester) benzoic acid, 4,4-((6-(((1,1-dimethylethyl)amino)-carbonyl)phenyl)amino)-1,3,5-triazine-2,4-diyl)diimino)bis,bis(2-ethylhexyl)ester), 3-(4-methylbenzylidene)-D,L-camphor (4-methylbenzylidene camphor), benzylidene-camphor-sulfonic acid, octocrylene, polyacrylamidomethyl-benzylidene-camphor, 2-ethylhexyl salicylate (octyl salicylate), 4-dimethyl-aminobenzoeacidethyl-2-hexylester (octyl dimethyl PABA), PEG-25 PABA, 2 hydroxy-4-methoxybenzo-phenone-5-sulfonic acid (5-benzophenone) and the sodium salt thereof, 2,2'-methylene-bis-6-(2H-benzotriazol-2-yl)-4-(tetramethyl-butyl)-1,1,3,3-phenol, the sodium salt of 2-2'-bis-(1,4-phenylene)1H-benzimidazole-4,6-disulfonic acid, (1,3,5)-triazine-2,4-bis((4-(2-ethyl-hexyloxy)-2-hydroxy)-phenyl)-6-(4-methoxyphenyl), 2-ethylhexyl-2-cyano-3,3-diphenyl-2-propenoate, glyceryl octanoate, di-p-methoxy cinnamic acid, p-amino-benzoic acid and its ester, 4-tert-butyl-4'-methoxydibenzoylmethane, 4-(2-β-glucopyranoxy)propoxy-2-hydroxybenzophenone, octyl salicylate, methyl-2,5-diisopropyl cinnamic acid, cinoxate, dihydroxy-dimethoxybenzophenone, disodium salts of 2,2'-dihydroxy-4,4'-dimethoxy-5,5'-disulfobenzophenone, dihydroxybenzophenone, 1,3,4-dimethoxyphenyl-4,4-dimethyl-1,3-pentanedione, 2-ethylhexyl-dimethoxybenzyliden-dioxoimidazolidinpropionate, methylene-bis-benztriazolyl tetramethylbutylphenol, phenyldibenzimidazoltetrasulfonate, bis-ethylhexyloxyphenol-methoxyphenol-triazine, tetrahydroxybenzophenone, terephthalylidendicamphor-sulfonic acid, 2,4,6-tris[4,2-ethylhexyloxycarbonyl)anilino]-1,3,5-triazine, methyl-bis(trimethylsiloxy)silyl-isopentyl trimethoxy cinnamic acid, amyl-p-dimethylaminobenzoate, amyl-p-dimethylamino benzoate, 2-ethylhexyl-p-dimethylaminobenzoate, isopropyl-p-methoxy cinnamic acid/diisopropyl cinnamic acid ester, 2-ethylhexyl-p-methoxy cinnamic acid, 2-hydroxy-4-methoxy benzophenone, 2-hydroxy-4-methoxybenzophenone-5-sulfonic acid and the trihydrate, 2-hydroxy-4-methoxybenzophenone-5-sulfonate sodium salt, phenyl-benzimidazole sulfonic acid, and combinations thereof. In at least one embodiment, the composition comprises from 0.001 wt.-% to 10 wt.-%, preferably from 0.05 wt.-% to 5 wt.-%, even more preferably from 0.1 wt.-% to 3 wt.-%, most preferably from 0.05 wt.-% to 1 wt.-% sun protection agent and/or UV filter. In at least one embodiment, the composition comprises a photoprotective substance in an amount of from 0.01 to 10 wt.-%, or from 0.1 to 5 wt.-%, particularly preferably from 0.2 to 2 wt.-%. The photoprotective substances include, in particular, all of the photoprotective substances specified in EP 1 084 696, which is incorporated herein by reference. In a preferred embodiment, the photoprotective substance is selected from the group consisting of 2-ethylhexyl 4-methoxycinnamate, methyl methoxycinnamate, 2-hydroxy-4-methoxybenzophenone-5-sulfonic acid, polyethoxylated p-aminobenzoates, and combinations thereof.

In at least one embodiment, the composition comprises an anti-oxidant. In at least one embodiment, the anti-oxidant is selected from the group consisting of amino acids, peptides, sugars, imidazoles, carotinoids, carotenes, chlorogenic acid, lipoic acid, thiols, thiol glycosyl esters, thiol N-acetyl esters, thiol methyl esters, thiol ethyl esters, thiol propyl esters, thiol amyl esters, thiol butyl esters, thiol lauryl esters, thiol palmitoyl esters, thiol oleyl esters, thiol linoleyl esters, thiol cholesteryl esters, thiol glyceryl esters, dilaurylthiodipropionate, distearylthiodipropionate, thiodipropionic acid, metal chelators, hydroxy acids, fatty acids, folic acids, vitamin C, tocopherol, vitamin A, stilbenes, derivatives and combinations thereof. In at least one embodiment, the anti-oxidant is selected from the group consisting of glycine, histidine, tyrosine, tryptophan, urocaninic acid, D,L-carnosine, D-carnosine, L-carnosine, beta-carotene, alpha-carotene, lycopene, dihydrolipoic acid, aurothioglucose, propylthiouracil, thioredoxine, glutathione, cysteine, cystine, cystamine, buthioninsulfoximine, homocysteinsulfoximine, buthioninsulfone, penta-, hexa-, heptathioninsulfoximine, hydroxyfatty acids, palmitic acid, phytinic acid, lactoferrin, citric acid, lactic acid, malic acid, humic acid, bile acid, bilirubin, biliverdin, EDTA, EGTA, linoleic acid, linolenic acid, oleic acid, butylhydroxyanisol, trihydroxybutyrophenone, ubichinon, ubichinol, ascorbylpalmitate, Mg-ascorbylphosphate, ascorbylacetate, vitamin E acetate, vitamin A palmitate, carnosine, mannose, ZnO, ZnSO₄, selenium methionine, stilbenes, superoxide dismutase, and combinations thereof. In at least one embodiment, the antioxidant is selected from the group consisting of vitamin A, vitamin A derivatives, vitamin E, vitamin E derivatives, and combinations thereof. In at least one embodiment, the composition comprises from 0.001 wt.-% to 10 wt.-%, preferably from 0.05 wt.-% to 5 wt.-%, even more preferably from 0.1 wt.-% to 3 wt.-%, most preferably from 0.05 wt.-% to 1 wt.-% antioxidant.

In at least one embodiment, the composition comprises a dye or pigment. In at least one embodiment, the composition comprises at least one pigment. Suitable dyes and pigments are disclosed in WO2013017262A1 in the table spanning pages 36 to 43. These may be colored pigments which impart color effects to the product mass or to hair, or they may be luster effect pigments which impart luster effects to the product mass or to the hair. The color or luster effects on the hair are preferably temporary, i.e. they last until the next hair wash and can be removed again by washing the hair with customary shampoos. In at least one embodiment, the composition comprises a total amount of from 0.01 wt.-% to 25 wt.-%, preferably from 5 wt.-% to 15 wt.-% pigment. In at least one embodiment, the particle size of the pigment is from 1 micron to 200 micron, preferably from 3 micron to 150 micron, more preferably 10 micron to 100 micron. The pigments are colorants which are virtually insoluble in the application medium, and may be inorganic or organic. Inorganic-organic mixed pigments are also possible. Preference is given to inorganic pigments. The advantage of inorganic pigments is their excellent resistance to light, weather and temperature. The inorganic pigments may be of natural origin. In at least one embodiment, the inorganic pigment is selected from the group consisting of chalk, ochre, umber, green earth, burnt sienna, graphite, and combinations thereof. The pigments may be white pigments, such as, for example, titanium dioxide or zinc oxide, black pigments, such as, for example, iron oxide black, colored pigments, such as, for example, ultramarine or iron oxide red, lustre pigments, metal effect pigments, pearlescent pigments, and fluorescent or phosphorescent pigments, where preferably at least one pigment is a colored, nonwhite pigment. In at least one embodiment, the pigment is selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze pigments), and combinations thereof. In at least one embodiment, the pigment is selected from the group consisting of titanium dioxide (CI 77891), black iron oxide (CI 77499), yellow iron oxide (CI 77492), red and brown iron oxide (CI 77491), manganese violet (CI 77742), ultramarine (sodium aluminum sulfosilicates, CI 77007, Pigment Blue 29), chromium oxide hydrate (CI 77289), Prussian blue (ferric ferrocyanide, CI 77510), carmine (cochineal), and combinations thereof. In at least one embodiment, the pigment is selected from the group consisting of pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, carmine etc. and where the color can be determined by varying the layer thickness. Such pigments are sold, for example, under the trade names Rona^{®}, Colorona^{®}, Dichrona^{®} and Timiron^{®} by Merck, Germany. In at least one embodiment, the pigment is selected from the group consisting of organic pigments such as sepia, gamboge, bone charcoal, Cassel brown, indigo, chlorophyll and other plant pigments. In at least one embodiment, the pigment is selected from the group consisting of synthetic organic pigments such as azo pigments, anthraquinoids, indigoids, dioxazine, quinacridone, phthalocyanine, isoindolinone, perylene and perinone, metal complex, alkali blue and diketopyrrolopyrrole pigments.

In at least one embodiment, the composition comprises from 0.01 wt.-% to 10 wt.-%, preferably from 0.05 wt.-% to 5 wt.-%, of at least one particulate substance. Suitable substances are, for example, substances which are solid at room temperature (25 °C) and are in the form of particles. In at least one embodiment, the particulate substance is selected from the group consisting of silica, silicates, aluminates, clay earths, mica, insoluble salts, in particular insoluble inorganic metal salts, metal oxides, e.g. titanium dioxide, minerals and insoluble polymer particles are suitable. The particles are present in the composition in undissolved, preferably stably dispersed form, and, following application to the keratin substrate and evaporation of the solvent, can deposit on the substrate in solid form. A stable dispersion can be achieved by providing the composition with a yield point which is large enough to prevent the solid particles from sinking. An adequate yield point can be established using suitable gel formers in a suitable amount. In at least one embodiment, the particulate substance is selected from the group consisting of silica (silica gel, silicon dioxide) and metal salts, in particular inorganic metal salts, where silica is particularly preferred. Metal salts are, for example, alkali metal or alkaline earth metal halides, such as sodium chloride or potassium chloride; alkali metal or alkaline earth metal sulfates, such as sodium sulfate or magnesium sulfate.

In at least one embodiment, the composition comprises a direct dye. Preferred among the direct dyes are the following compounds, alone or in combination with one another: Hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydroxyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof. Particularly preferred among the aforesaid direct dyes are the following compounds, alone or in combination with one another: hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydro-xyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)- amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof. In at least one embodiment, the total quantity of direct dyes in the composition amounts to 0.01 to 15 wt.-%, preferably 0.1 to 10 wt.-%, most preferred 0.5 to 8 wt.-%.

In at least one embodiment, the home care composition comprises at least one anionic surfactant. In at least one embodiment, the home care composition comprises at least one nonionic surfactant. In at least one embodiment, the home care composition comprises at least one anionic surfactant and at least one nonionic surfactant. Suitable anionic and nonionic surfactants are described further above.

In a preferred embodiment, the home care composition is an automatic dishwashing product. In a preferred embodiment, the home care composition is an automatic dishwashing product which comprises at least one alkylene oxide-based surfactant. In a preferred embodiment, the home care composition is an automatic dishwashing product which comprises at least one of an ethylene oxide-based surfactant, ethylene/propylene oxide-based surfactant, ethylene/butylene oxide-based surfactant, or ethylene/pentylene oxide-based surfactant. In a preferred embodiment, the home care composition is an automatic dishwashing product which comprises at least one fatty alcohol alkoxylate, for example at least one fatty alcohol ethoxylate, fatty alcohol ethoxylate/propoxylate, fatty alcohol ethoxylate/butoxylate or fatty alcohol ethoxylate/pentoxylate.

In a preferred embodiment, the home care composition is a laundry detergent. In a preferred embodiment, the home care composition is a laundry detergent which comprises at least one of linear alkylbenzene sulfonates (LABS), sodium lauryl ether sulfate (SLES), sodium lauryl sulfate (SLS), soap, Laureth-3, Laureth-9, Laureth-7, alkyl polyglycoside (preferably alkyl polyglucoside) surfactants (APG), or glucamide surfactants.

The invention is further illustrated by the following examples, without being limited thereby.

### Examples

### Example 1:

The purpose of this study is to investigate the performance of dimethylglucamine in chelating iron(III) and copper(II) ions vs. known chelating molecules such as EDTA, Piroctone Olamine, Glycine, Triethanolamine and Aminomethylpropanol.

### a) Test product:

The following substances have been used as product for testing chelation of Iron (III) and Copper (II) ions: Dimethylglucamine, Piroctone Olamine, EDTA, Glycine, Aminomethylpropanol and Triethanolamine.

### b) Complexing ability test vs. EDTA:

The relative complexing ability of the chelant is evaluated vs. EDTA by observing color changes and precipitate formation.

To Fe³⁺ and Cu²⁺ solutions (c = 0,2 mmol/L), solutions of chelants are added:
- Na₄EDTA at 0,4 mmol/L
- Na₄EDTA at 2 mmol/L
- Only chelant (Dimethylglucamine or Piroctone Olamine) at 0,6 mmol/L
- Chelant (Dimethylglucamine or Piroctone Olamine) at 0,6 mmol/L + Na₄EDTA 0,4 mmol/L
- Chelant (Dimethylglucamine or Piroctone Olamine) at 0,6 mmol/L + Na₄EDTA 2 mmol/L

### c) Confirmation of complex formation with chelants similar to Dimethylglucamine:

To Fe³⁺ and Cu²⁺ solutions (c = 0,2 mmol/L), solutions of single chelants (Dimethylglucamine, Piroctone Olamine, Glycine, Triethanolamine (TEA), Aminomethylpropanol (AMP)) are added at concentration of c=0,2 mmol/L. The relative complexing ability of the chelant is evaluated by observing color changes and precipitate formation.

### d) Complexing ability test vs. Aminomethylpropanol:

The relative complexing ability of the chelant Dimethylglucamine is evaluated vs. Aminomethylpropanol by observing color changes and precipitate formation. In particular Aminomethylpropanol was chosen since in the previous test c) it was shown that Aminomethylpropanol makes complex with Copper that differs in color of Copper complex with Dimethylglucamine. Therefore, it is possible to evaluate the strength of complexation of Aminomethylpropanol and Dimethylglucamine when both chelants are present in solution of Copper²⁺ ions by observing the color of solution. To Cu²⁺ solutions (c = 0,2 mmol/L), solutions of chelants are added:
- Aminomethylpropanol at 0,6 mmol/L
- Dimethylglucamine at 0,6 mmol/L
- Aminomethylpropanol at 0,6 mmol/L + Dimethylglucamine 0,6 mmol/L

### e) Reagents:

| | |
|---|---|
| Fe³⁺: | 0,2 mmol/L solution |
| Cu²⁺: | 0,2 mmol/L solution |
| Na₄EDTA: | 0,4 mmol/L solution |
| Na₄EDTA: | 2 mmol/L solution |
| Dimethylglucamine: | 0,6 mmol/L solution |
| Piroctone Olamine: | 0,6 mmol/L solution |
| Glycine: | 0,6 mmol/L solution |
| Triethanolamine: | 0,6 mmol/L solution |
| Aminomethylpropanol: | 0,6 mmol/L solution |

### f) Results

### f1) Complexing ability test vs. EDTA (Test b)

| Chelant | Result |
|---|---|
| 0.4 mmol/L EDTA + Piroctone Olamine | Turbid intense yellow-orange color solution |
| 2 mmol/L EDTA + Piroctone Olamine | Clear solution with distinct orange precipitate |
| Piroctone Olamine | Turbid yellowish solution with distinct orange precipitate |
| 0.4 mmol/L EDTA | Clear, very slightly yellowish solution |
| 2 mmol/L EDTA | Clear, very slightly yellowish solution |

Piroctone Olamine is very strong chelating agent for Iron³⁺ ions and in presence of well-established chelant EDTA, Piroctone Olamine is stronger than EDTA since distinct orange complex of Iron ³⁺ and Piroctone Olamine is formed.

| Chelant | Result |
|---|---|
| 0.4 mmol/L EDTA + DMG | Clear yellow-orange solution |
| 2 mmol/L EDTA + DMG | Clear yellow-orange solution |
| DMG | Clear solution with distinct orange precipitate |
| 0.4 mmol/L EDTA | Clear, very slightly yellowish solution |
| 2 mmol/L EDTA | Clear, very slightly yellowish solution |

Dimethylglucamine (DMG) is strong chelating agent for Iron³⁺ ions and Dimethylglucamine is stronger chelating agent than EDTA which is shown in trials where both EDTA and Dimethylglucamine are present and distinct colored complex of Iron³⁺ and Dimethylglucamine is formed.

### f2) Confirmation of complex formation with chelants similar to Dimethylglucamine (Test c)

**Table: Complex formation with Fe³⁺ ions**

| Chelant | pH | Result |
|---|---|---|
| Dimethylglucamine | 3.4 | Clear solution with distinct orange precipitate |
| EDTA | 10.6 | Clear, very slightly yellowish solution |
| Glycine | 3.3 | Clear solution with distinct orange precipitate |
| Piroctone Olamine | 3.8 | Turbid solution with distinct orange precipitate |
| Aminomethylpropanol | 4.0 | Clear solution with distinct orange precipitate |
| Triethanolamine | 3.9 | Clear solution with distinct orange precipitate |

**Table: Complex formation with Cu²⁺ ions**

| Chelant | pH | Result |
|---|---|---|
| Dimethylglucamine | 6.5 | Clear, light blue solution |
| EDTA | 10.0 | Clear, light blue solution |
| Glycine | 4.6 | Clear, slightly bluish solution |
| Piroctone Olamine | 8.3 | Turbid, light blue solution |
| Aminomethylpropanol | 9.2 | Clear solution with distinct brown precipitate |
| Triethanolamine | 7.5 | Clear, light blue solution |

Colored complexes of different chelants are formed with Iron³⁺ and Copper²⁺ ions. Dimethylglucamine forms distinct orange complexes with Iron³⁺ ions and blue complexes with Copper²⁺ ions.

### f3) Complexing ability test of Cu²⁺ ions with Dimethylglucamine vs. Aminomethylpropanol (Test d)

| Chelant | Result |
|---|---|
| 0.6 mmol/L AMP + 0.6 mmol/L DMG | Clear light blue solution |
| 0.6 mmol/L AMP | Clear solution with distinct brown precipitate |
| 0.6 mmol/L DMG | Clear light blue solution |

Dimethylglucamine (DMG) is very strong chelating agent and in presence of Aminomethylpropanol (AMP) and Cu²⁺ ions distinct blue complex of Dimethylglucamine is formed instead of brown complex of Aminomethylpropanol and Cu²⁺ ions.

### Example 2: Testing Dimethylglucamine in shampoo application

Water containing 0.01% of Iron (III) Sulfate was prepared and used for basic shampoo formulation. It was prepared first formulation without any chelating agent (S0). Blank formulation (S0) is yellow. In following trials were tested EDTA (S1), Piroctone Olamine (S2), Dimethylglucamine (S3) and Glycine (S4) respectively.

| Shampoo | S0 | S1 | S2 | S3 | S4 |
|---|---|---|---|---|---|
| Genapol LRO liquid (Sodium Laureth Sulfate) | 40 | 40 | 40 | 40 | 40 |
| Deionized water with added 0.01% of Iron (III) Sulfate | 51 | 51 | 51 | 51 | 51 |
| Genagen KB (Coco Betaine) | 6 | 6 | 6 | 6 | 6 |
| Genapol LA 030 (Laureth-3) | 2 | 2 | 2 | 2 | 2 |
| EDTA | | 0.25 | | | |
| Octopirox (Piroctone Olamine) | | | 0.25 | | |
| NeutroTain DMG (Dimethylglucamine) | | | | 0.5 | |
| Glycine | | | | | 0.25 |
| pH value | 4.9 | 6.1 | 5.8 | 7.2 | 4.9 |
| adjusted pH | 5.3 | 5.3 | 5.2 | 5.2 | 5.3 |
| NaCl | 0.5 | 0.5 | 0.5 | 0.5 | 0.5 |
| viscosity | 2900 | 4000 | 7400 | 2200 | 2700 |

The trial S3 with Dimethylglucamine has less yellow color than the blank trial S0 (no chelating agents). This means that complexes of DMG and Iron will not deteriorate the color of final shampoo and the result is very similar to shampoo where EDTA (S1) was used. Trials with Piroctone Olamine (S2) and Glycine (S4) have strong yellow or orange color. It is a good indication for DMG to be used in hair shampoos as chelating agent without causing discolorations.

Tap water containing small amounts (some ppm) of iron ions was used to prepare basic shampoo formulation. It was prepared first formulation without any chelating agent (S0). In following trials were tested EDTA (S1), Piroctone Olamine (S2), Dimethylglucamine (S3) and Glycine (S4).

| Shampoo | S0 | S1 | S2 | S3 | S4 |
|---|---|---|---|---|---|
| Genapol LRO liquid (Sodium Laureth Sulfate) | 40 | 40 | 40 | 40 | 40 |
| Tap water | 51 | 51 | 51 | 51 | 51 |
| Genagen KB (Coco Betaine) | 6 | 6 | 6 | 6 | 6 |
| Genapol LA 030 (Laureth-3) | 2 | 2 | 2 | 2 | 2 |
| EDTA | | 0.25 | | | |
| Octopirox (Piroctone Olamine) | | | 0.25 | | |
| NeutroTain DMG (Dimethylglucamine) | | | | 0.5 | |
| Glycine | | | | | 0.25 |
| pH value | 5 | 5.3 | 6.2 | 8.4 | 5 |
| adjusted pH | 5.2 | 5.3 | 5.3 | 5.2 | 5.3 |
| NaCl | 0.5 | 0.5 | 0.5 | 0.5 | 0.5 |
| viscosity | 3000 | 5500 | 7700 | 4000 | 3300 |

The trial S3 with Dimethylglucamine has less yellow color than the blank trial S0 (no chelating agents). This means that complexes of DMG and Iron will not deteriorate the color of final shampoo and the result is very similar to shampoo where EDTA (S1) was used. Trials with Piroctone Olamine (S2) and Glycine (S4) have slightly yellow color. It is a good indication for DMG to be used in hair shampoos as chelating agent without causing discolorations.

### Conclusion

The study confirmed chelation ability of Dimethylglucamine to make stable complexes with Fe³⁺ and Cu²⁺ ions. This can allow usage of Dimethylglucamine as efficient chelating agent in different cosmetic applications where neutralization of iron and copper ions is fundamental to achieve the highest performance of cosmetic formulation.

### Examples 3 to 24: Further example compositions

If not indicated otherwise, % refers to % by weight.

### Example 3: Liquid soap / body wash

| Ingredient | INCI | 1 %WT | 2 %WT | 3 %WT | 4 %WT | 5 %WT | Function |
|---|---|---|---|---|---|---|---|
| Hostapon SCI 85 P | Sodium Cocoyl Isethionate (85%) | 8.21 | 8.21 | 8.21 | 8.21 | 5 | Primary Surfactant |
| Parfum | | 0.3 | 0.3 | 0.3 | 0.3 | 0.3 | Fragrance |
| Water | Aqua | add to 100 | add to 100 | add to 100 | add to 100 | add to 100 | |
| Genamin PQ 43 PB | Polyquaternium-43 | 0.3 | 0.3 | 0.3 | 0.3 | 0.3 | Conditioning Agent |
| Glucotain Care | Cocoyl Methyl Glucamide (35%) | 10.04 | 10.04 | 10.04 | 10.04 | 10.04 | Primary Surfactant |
| Hostapon SG | Sodium Cocoyl Glycinate (30%) | 3.29 | | | | | Secondary Surfactant |
| Hostapon SGN | Sodium Cocoyl Glutamate (33%) | | 3.29 | | | 10 | Secondary Surfactant |
| Genagen KB | Coco-Betaine (30%) | | | 3.29 | | | Secondary Surfactant |
| Hostapon CT Paste | Sodium Methyl Cocoyl Taurate (30%) | | | | 3.29 | | Secondary Surfactant |
| NeutroTain DMG | Dimethylglucamine | 0.1 | 0.1 | 0.1 | 0.1 | 0.1 | Chelating Agent |
| Sodium Benzoate | Sodium Benzoate | 0.3 | 0.3 | 0.3 | 0.3 | 0.3 | Preservative |
| Perlogen SF 3000 | Aqua (and) Glycol Distearate (and) Laureth-4 (and) Cocamidopropyl Betaine | 0.5 | 0.5 | 0.5 | 0.5 | 0.5 | Pearlizing Agent |
| Thickener | Thickener | qs | qs | qs | qs | qs | Viscosity Adjustment |

### Example 4: Cleansing Stick

| Ingredient | INCI | [%] |
|---|---|---|
| Water | Aqua (Water) | ad 100,0 |
| Glycerin | Glycerin | 8 |
| NeutroTain DMG | Dimethylglucamine | 0.1 |
| Hostapon SCI 85 C | Sodium Cocoyl Isethionate | 10.25 |
| Plantasens Olive LD | Hydrgenated Ethylhexyl Olivate (and) Hydrogenated Olive Oil Unsaponifiables | 12 |
| Plantasens Carefeel Light | Ethylhexyl Olivate | 12 |
| Plantasens Cosmetic Wax A4 | Hydrogenated Vegetable Oil | 15 |
| Plantasens Emulsifier HE20 | Cetearyl Glucoside, Sorbitan Olivate | 11.25 |
| Nipaguard SCP | Phenoxyethanol, Sorbitan Caprylate | 0.75 |
| Essential oil blends | Essential oils | 0.5 |
| Mixed Tocopherols | Tocopherol | 0.5 |

### Example 5: Cleansing Soap Bar

| Ingredient | INCI | [%] |
|---|---|---|
| Soap Base Opal 508 SG | Sodium Palmate, Sodium Palm Kernelate, Water, Glycerin, Pentasodium Pentatate, EDTA | add to 100 |
| NeutroTain DMG | Dimethylglucamine | 0.10 |
| GlucoTain^{®} Sense | Sunfloweroyl Methyl Glucamide | 1.00 |
| CareMag^{®} D | Magnesium Hydroxide (and) Magnesium Carbonate Hydroxide | 2.50 |
| Velsan^{®} CGE | Caprylyl Glyceryl Ether | 1.00 |
| Fragrance Golden Temptation (Cosnaderm) | Fragrance | 0.40 |
| Color Clormix blue CI 42090, CI 16185 | Dye | 0.05 |

### Example 6: Solid Shampoo Bar

| Ingredient | INCI | Function | % |
|---|---|---|---|
| Medialan LD PF 10 | Sodium Lauroyl Sarcosinate | Surfactant | 10.00 |
| Hostapon SCI 85 G | Sodium Cocoyl Isethionate | Surfactant | 37.00 |
| Glucotain Care | Cocoyl Methyl Glucamide | Surfactant | 20.00 |
| Polyglykol 8000 | PEG-180 | Binder | 27.00 |
| Genadvance Repair | Quaternium-98 | Hair conditioning agent | 3.00 |
| NeutroTain DMG | Dimethylglucamine | Chelating agent | 0.10 |
| Nipaguard SCE | Sorbitan Caprylate (and) Propanediol (and) Benzoic Acid | Preservative | 1.00 |
| Perfume | Fragrance | Fragrance | 0.50 |

### Example 7: Hair & Skin Lotion

| Phase | Ingredient | Function | As is |
|---|---|---|---|
| A | Water | Solvent | ad 100 % |
| | NeutroTain DMG | Chelating agent | 0.10 |
| | Dimethylglucamine | | |
| B | Dongbaek (Tsubaki) Oil | Anti-Ageing Active | 2.00 % |
| | Camellia Japonica Seed Oil | | |
| | Plantasens Olive LD | Emollient | 0.05 |
| | Hydrogenated Ethylhexyl Olivate (and) | | |
| | Hydrogenated Olive Oil Unsaponifiables | | |
| | Genadvance^{®} Hydra Lauryl/Myristyl Polyricinoleate (and) Glycerin | Hair Moisturizer, Hair Conditioner | 1.50 % |
| C | Aristoflex^{®} Silk | Rheology Modifier | 0.70 % |
| | Sodium Polyacryloyldimethyl Taurate | | |
| D | Herbex Yeast Beta Glucan | Skin Well-Being Enhancer, UV Protector | 2.00 % |
| | Butylene Glycol, Yeast Beta Glucan | | |
| | Plantasens^{®} Berto^{™} Orthomoist | Skin Moisturizer | 1.00 % |
| | Orthosiphon Stamineus Leaf Extract (and) Chlorphenesin | | |
| E | Nipaguard^{®} EHP | Preservative | 1.00 % |
| | Ethylhexylglycerin, Phenoxyethanol | | |
| | Fragrance | Fragrance | 0.40 % |
| F | Sodium Hydroxide 10% soln. (pH:4,5-5,5) | pH Adjuster | q.s. |

### Example 8: Hair & Skin Cream

| | Ingredient | Function | As is [%] |
|---|---|---|---|
| A | Water | Solvent | ad 100 |
| | NeutroTain DMG | Chelating agent | 0.01 |
| | Dimethylglucamine | | |
| B | Plantasens^{®} Sunflower Butter | Film Former | 4.00 |
| | Helianthus Annuus (Sunflower) Seed Oil (and) Hydrogenated Vegetable Oil | | |
| | Plantasens^{®} Emulsifier HP 10 | Emulsifier | 3.00 |
| | Sucrose Polystearate, Cetearyl Alcohol, Olea Europaea (Olive) Oil Unsaponifiables | | |
| | Genadvance^{®} Hydra | Hair Moisturizer, Hair Conditioner | 1.50 |
| | Lauryl/Myristyl Polyricinoleate (and) Glycerin | | |
| C | Aristoflex^{®} BLV | Rheology Modifier | 0.70 |
| | Ammonium Acryloyldimethyltauratel Beheneth-25 Methacrylate Crosspolymer | | |
| | Aristolex^{®} AVC | Rheology Modifier | 0.30 |
| | Ammonium Acryloyldimethyltaurate/VP Copolymer | | |
| D | Herbex Yeast Beta Glucan | Skin Well-Being Enhancer, UV Protector | 2.00 |
| | Butylene Glycol, Yeast Beta Glucan | | |
| | RedSnow^{®} | Anti-Ageing Active | 2.00 |
| | Propanediol, Water, Camellia Japonica Flower Extract | | |
| | Plantasens^{®} Berto^{™} Orthomoist | Skin Moisturizer | 1.00 |
| | Orthosiphon Stamineus Leaf Extract (and) Chlorphenesin | | |
| E | Nipaguard^{®} EHP | Preservative | 1.00 |
| | Ethylhexylglycerin, Phenoxyethanol | | |
| | Fragrance | Fragrance | 0.40 |
| F | Sodium Hydroxide 10% soln. (pH:4,5-5,5) | pH Adjuster | q.s. |

### Example 9: Sun Cream SPF 50+, PA++++ (Calculated)

| Phase | Ingredient | INCl | Function | As is [%] |
|---|---|---|---|---|
| A | Water | Water | Diluent | ad 100 |
| | NeutroTain DMG | Dimethylglucamine | Chelating agent | 0.10 |
| | Glycerin | Glycerin | Humectant | 3.00 |
| | Eclipsogen^{®} PBSA | Phenylbenzimidazole Sulfonic Acid | UV Filter | 2.00 |
| | Sodium Hydroxide | Sodium Hydroxide | pH Adjuster | q.s. to pH 7 |
| B | Hostaphat^{®} KL 340 D | Trilaureth-4 Phosphate | Emulsifier | 0.75 |
| | Cetearyl Alcohol | Cetearyl Alcohol | Stabilizer | 2.25 |
| | Plantasens^{®} Carefeel Light | Ethylhexyl Olivate | Emollient | 6.00 |
| | Eclipsogen^{®} Sorb S | Bis-Ethylhexyloxyphenol Methoxyphenyl Triazine | UV Filter | 5.00 |
| | Eclipsogen^{®} OC | Octocrylene | UV Filter | 6.00 |
| | Eclipsogen^{®} EHT | Ethylhexyl Triazone | UV Filter | 3.00 |
| C | Eclipsogen^{®} Sorb M | Methylene Bis-Benzotriazolyl Tetramethylbutylphenol (and) Water (and) Decyl Glucoside (and) Xanthan Gum (and) Propylene Glycol | UV Filter | 10.00 |
| D | Aristoflex^{®} Silk | Sodium Polyacryloyldimethyl Taurate | Rheology Modifier | 1.50 |
| E | Nipaguard^{®} PO5 | Phenoxyethanol (and) Piroctone Olamine | Preservative | 1.00 |
| | Perfume | | Fragrance | 0.30 |

### Example 10: Hand Hygiene wipes

| Phase | Ingredient | INCl | *w*/*w%* |
|---|---|---|---|
| A | Water | Aqua | To 100% |
| | NeutroTain DMG | Dimethylglucamine | 0.1 |
| | Polyglykol 400 | PEG-8 | 2.0 |
| | Genapol G260 | Glycereth-26 | 2.0 |
| B | Glucotain Sense | Sunfloweroyl methyl glucamide | 0.5 |
| B | Emulsogen HCO 040 | PEG-40 Hydrogenated castor oil | 1.0 |
| | Fragrance | Fragrance | 0.2 |
| C | Benzethonium Chloride | Benzethonium Chloride | 0.1 |

### Example 11: Facial Cleansing wipes

| Phase | Ingredient | INCI | % (w/w) |
|---|---|---|---|
| A | Water | Water | to 100 |
| | NeutroTain DMG | Dimethylglucamine | 0.10 |
| A | Panthenol | Panthenol | 0.10 |
| A | Aristoflex TAC | Ammonium Acrylyol Dimethyl Taurate/ Carboxyethyl Acrylate Crosspolymer | 0.10 |
| B | Plantasens Emulsifier CCT | Caprylic/Capric Triglyceride, Aqua, Lauryl Glucoside, Glycerin, Sodium Lauroyl Lactylate, Glyceryl Stearate, Cetearyl Alcohol, Sodium Stearoyl Lactylate | 2.50 |
| B | Plantasens LS⁶ | Olea europea Olive oil, Simmondsia Chinensis seed oil, Oleic acid, Squalene, Tocopherol, Helianthus Annuus (Sunflower) Seed oil | 0.05 |
| B | Tocopheryl Acetate | Tocopheryl Acetate | 0.05 |
| B | Nipaguard SCE | Sorbitan Caprylate, Propanediol, Benzoic Acid | 1.50 |
| C | Fragrance | Fragrance | 0.02 |

### Example 12: Deodorant roll-on

| Phase | Ingredient | INCl | Function | A [%] | B [%] |
|---|---|---|---|---|---|
| A | Water | Aqua | Solvent | to 100.00 | to 100.00 |
| | NeutroTain DMG | Dimethylglucamine | Chelating agent | 0.1 | 0.1 |
| | Aristoflex^{®} Silk | Sodium Acryloyldimethyltaurate | Rheology Modifier, Sensory enhancer | | 0,30 |
| | Xanthan Gum FNCSP-PC | Xanthan Gum | Rheology Modifier | 0.80 | 0,50 |
| B | GlucoTain^{®} Sense | *Sunfloweroyl Methylglucamide* | Spreading Agent | 2,00 | 2,00 |
| | Velsan^{®} CGE | Caprylyl Glyceryl Ether | Preservative booster, Emollient | 1,00 | 1,00 |
| C | Water | | Solvent | 20,00 | 20,00 |
| | CareMag^{®} D | *Magnesium Hydroxide (and) Magnesium Hydroxide Carbonate* | Deodorant Active, Texturizer | 5,00 | 7,50 |
| | Corn Starch | Zea Mays (Corn) Starch | Absorber, Texturizer | 3,00 | |
| D | Fragrance "Aquasol Unity" (Bell & Fragrance) | Fragrance | Fragrance | 1,00 | 1,00 |

### Example 13: Deodorant Stick

| | Ingredient | INCl | Function | [%] |
|---|---|---|---|---|
| A | CareMag^{™} D | Magnesium Hydroxide (and) Magnesium Hydroxide Carbonate | Deodorant Active | 20,00 |
| | Reisita Natural 9094 | Oryza Sativa (Rice) Starch | Bulking Agent, Texturizer | 10,00 |
| | Corn PO4 "B" 9042 | Distarch Phosphate | Bulking Agent, Texturizer | 8,00 |
| | Fragrance "Vanilla Blossom | | Fragrance | 1,00 |
| | Plantasens^{®} Vitamin E Natural 50 | Tocopherol (and) Helianthus Annuus (Sunflower) Seed Oil | Antioxidant | 1,00 |
| | MultiEx BSASM^{™} | Butylene Glycol (and) Aqua (and) Centella Asiatica Extract (and) Polygonum Cuspidatum Root Extract (and) Scutellaria Baicalensis Root Extract (and) Camellia Sinensis Leaf Extract (and) Glycyrrhiza Glabra (Licorice) Root Extract (and) Chamomilla Recutita (Matricaria) Flower Extract (and) Rosmarinus Officinalis (Rosemary) Leaf Extract | Soothing Active | 1,00 |
| | Velsan^{®} CGE | Caprylyl Glyceryl Ether | Preservation booster, emollient | 1,00 |
| B | Plantasens^{®} FLASH 80 | Dodecane (vegetable) | Emollient | 14,00 |
| | Plantasens^{®} Olive LS SP ECO | Coco-Caprylate/Caprate (and) Hydrogenated Olive Oil Unsaponifiables | Emollient | 4,60 |
| | Sunflower Seed Oil | Helianthus Annuus (Sunflower) Seed Oil | Emollient | 2,00 |
| | Hostacerin^{®} SFO | Sunflower Seed Oil Sorbitol Esters | Emulsifier | 2,00 |
| | Plantasens^{®} Babassu Butter | Orbignya Oleifera Seed Oil | Emollient | 6,20 |
| | Plantasens^{®} Cosmetic Wax A4 | Hydrogenated Vegetable Oil | Structuring agent | 4,00 |
| | Kahlwax 6607L (Sunflower Seed Wax) | Helianthus Annuus Seed Cera (and) Ascorbyl Palmitate (and) Tocopherol | Structuring agent | 15,00 |
| | Lanette^{®} O | Cetearyl Alcohol | Structuring agent | 10,20 |

### Example 14: Antiperspirant roll-on

| Phase | Ingredient | INCI | Function | [%] |
|---|---|---|---|---|
| A | Drygen L | Aluminum Chlorohydrate | Antiperspirant agent | 30.00 |
| | NeutroTain DMG | Dimethylglucamine | Chelating agent | 0.10 |
| | Water | Water | Solvent | ad 100.00 |
| | Polyglykol 400 | PEG-8 | Humectant | 3.00 |
| | Ethanol | Ethanol | Solvent | 17.00 |
| | Fragrance | Fragrance | Fragrance | 0.30 |
| B | Tylose H 4000 G4 | Hydroxyethylcellulose | Thickener | 2.50 |

### Example 15: Shampoo

| Ingredient | INCI | %WT | Function |
|---|---|---|---|
| Genapol LRO | Sodium Laureth Sulfate | 18.52 | Primary Surfactant |
| Parfum | | 0.3 | Fragrance |
| Water | Aqua | add to 100 | |
| PQ-7 | Polyquaternium-7 | 0.5 | Conditioning Agent |
| Glucotain Liquiflex | Lauroyl/Myristoyl Glucamide (and) Coco-Betaine | 8.57 | Primary Surfactant |
| NeutroTain DMG | Dimethylglucamine | 0.1 | Chelating Agent |
| Sodium Benzoate | Sodium Benzoate | 0.3 | Preservative |
| Perlogen SF 3000 | Aqua (and) Glycol Distearate (and) Laureth-4 (and) Cocamidopropyl Betaine | 0.5 | Pearlizing Agent |
| Sodium Chloride, Magnesium Sulfate, Sodium Lactate | Sodium Chloride, Magnesium Sulfate, Sodium Lactate | qs | Viscosity Adjustment |

### Example 16: Hair Mask

| Phase | Ingredient | INCI | Function | As is |
|---|---|---|---|---|
| A | Plantasens^{®} Natural Emulsifier CP5 | Glyceryl Oleate (and) Polyglyceryl-3-Polyricinoleate (and) Olea Europaea (Olive) Oil Unsaponifiables | Emulsifier | 1.00 |
| | Cetyl Alcohol | | Stabilizer | 4.00 |
| | Stearic Acid | | Stabilizer | 1.00 |
| | Plantasens^{®} Argan Butter | Argania Spinosa Kernel Oil (and) Hydrogenated Vegetable Oil | Emollient | 1.00 |
| | Plantasens^{®} Apricot Butter | Prunus Armeniaca (Apricot) Kernel Oil (and) Hydrogenated Vegetable Oil | Emollient | 0.50 |
| B | Water | Water | Diluent | ad 100.0 |
| | NeutroTain DMG | Dimethylglucamine | Chelating agent | 0.10 |
| | Allantoin | Allantoin | Active | 0.10 |
| | Tylose H100070 NP2 | Hydroxyethylcellulose | Rheology Modifier | 0.50 |
| | Genamin^{®} CTAC | Cetrimonium Chloride | Conditioning Agent | 0.50 |
| | Genamin^{®} KDMP | Behentrimonium Chloride | Conditioning Agent | 1.50 |
| | Glycerol | | Humectant | 5.00 |
| C | Beraclay Red | Magnesium Aluminum Silicate/ Kaolin/ Mica | Absorbing agent | 2.00 |
| | Beraclay Dark Red (Beraca) | Magnesium Aluminum Silicate/ Kaolin/ Montmorillonite / Mica | Absorbing agent | 4.00 |
| | ImerCare^{™} 04K | Kaolin | Absorbing agent | 12.00 |
| D | Plantasens^{®} Lime Serum (Clariant) | Citrus Limon (Lemon) Seed Oil (and) Phytosterols (and) Olea Europaea (Olive) Oil Unsaponifiables (and) Beeswax | Emollient | 0.30 |
| | Plantasens^{®} Olive LD (Clariant) | Hydrogenated Ethylhexyl Olivate (and) Hydrogenated Olive Oil Unsaponifiables | Emollient | 0.50 |
| | Fragrance 'Orchidee' (Cosnaderm) | | Fragrance | 0.50 |
| E | EMortal^{™} Pep (BioSpectrum) | Propanediol (and) Water (and) Pisum Sativum (Pea) Peptide | Active | 0.80 |
| | COSI-PLANT Green Tea GW (Cosnaderm) | Glycerin, Aqua, Camellia Sinensis Leaf Extract | Active | 0.30 |
| | Tocopheryl Acetate | | Active | 0.50 |
| F | Nipaguard^{®} SCE (Clariant) | Sorbitan Caprylate (and) Propanediol (and) Benzoic Acid | Preservative | 1.20 |
| | NeutroTain DMG | Dimethylglucamine | Neutralizing Agent | q.s. to pH approx. 4.30 |

### Example 17: Natural Hair Conditioner

| Phase | \| Ingredient | \| INCI | [%] |
|---|---|---|---|
| A | Plantasens^{®} Tara Gum | Caesalpinia spinosa gum | 0.70 |
| | Glycerin | Glycerin | 3.50 |
| | NeutroTain DMG | Dimethylglucamine | 1.00 |
| B | Water | water | add to 100% |
| C | Plantasens^{®} Olive LD | Hydrogenated Ethylhexyl Olivate (and) Hydrogenated Olive Oil Unsaponifiables | 5.00 |
| | Tsubaki Oil | Camellia Japonica Seed Oil | 2.00 |
| | Genadvance^{®} Life | Polyquaternium-116 | 2.00 |
| | Plantasens^{®} Emulsifier CP 40 | Polyglyceryl-3 Polyricinoleate (and) Sorbitan Oleate | 5.00 |
| D | Herbex Yeast Beta Glucan | Butylene Glycol, Yeast Beta Glucan | 2.00 |
| | Plantasens^{®} Berto Orthomoist | Orthosiphon Stamineus Leaf Extract (and) Chlorphenesin | 1.00 |
| | Nipaguard EHP | Ethylhexylglycerin | 1.00 |
| | Fragrance (Elderberry) | Fragrance | 0.10 |

### Example 18: Hair dye base

| | Ingredient | INCI | 1 | 2 | 3 | 4 |
|---|---|---|---|---|---|---|
| A | Laureth-4 | Laureth-4 | 4.00 | 4.00 | 4.00 | 4.00 |
| | Oleic Acid | Oleic Acid | 4.00 | 4.00 | 4.00 | 4.00 |
| | Oleyl Alcohol | Oleyl Alcohol | 4.00 | 4.00 | 4.00 | 4.00 |
| | Glycol Stearate | Glycol Stearate | 6.00 | 6.00 | 6.00 | 6.00 |
| | Glyceryl Stearate SE | Glyceryl Stearate SE | 5.00 | 5.00 | 5.00 | 5.00 |
| | Cetearyl Alcohol | Cetearyl Alcohol | 6.00 | 6.00 | 6.00 | 6.00 |
| | Polyquaternium-22 | Polyquaternium-22 | 1.00 | 1.00 | 1.00 | 1.00 |
| B | Water | Water | 25.00 | 25.00 | 25.00 | 25.00 |
| | Sodium Sulphite | Sodium Sulphite | 0.50 | 0.50 | 0.50 | 0.50 |
| | Ascorbic Acid | Ascorbic Acid | 0.50 | 0.50 | 0.50 | 0.50 |
| | NeutroTain DMG | NeutroTain DMG | 0.10 | 0.10 | 0.10 | 0.10 |
| | Colorant blend Dark Brown | Colorant blend Dark Brown | 2.00 | 2.00 | 2.00 | 2.00 |
| | Sodium Lauryl Sulfate | Sodium Lauryl Sulfate | 2.00 | 2.00 | 2.00 | 2.00 |
| C | Genapol LRO liquid | Sodium Laureth Sulfate 28% | 5.00 | 5.00 | 5.00 | 5.00 |
| | Propylene glycol | Propylene glycol | 10.00 | 10.00 | 10.00 | 10.00 |
| D | Ammonia 25% | Ammonia 25% | 7.00 | | | |
| | Dimethylglucamine 50% | Dimethylglucamine 50% | | 7.00 | | |
| | Monoethanolamine 99% | MEA 99% | | | 7.00 | |
| | Aminomethylpropanol 95% | | | | | 7.00 |
| | Water | Water | add to 100 | add to 100 | add to 100 | add to 100 |

### Example 19: Toothpaste

| Ingredient | INCI | %wt |
|---|---|---|
| Meritol 161 | Sorbitol, Aqua | 30.0 |
| Water | Aqua | 19.0 |
| NeutroTain DMG | Dimethylglucamine | 0.05-0.1 |
| Glycerin 99,5 Ph. Eu. | Glycerin | 15.0 |
| Keltrol CG-SFT | Xanthan Gum | 0.3 |
| Purolan E 171A | Titanium Dioxide | 1.0 |
| Zeodent 113 | Hydrated Silica | 12.0 |
| Zeodent 167 | Hydrated Silica | 13.0 |
| Surfactant | Surfactant | 4.0-6.0 |
| Aroma Toothpaste | Aroma Toothpaste | 0.8 |
| Citric acid sol 20% | Citric acid sol 20% | 2.5 |

### Example 20: Foundation

| Phase | Ingredient | INCI | [%] |
|---|---|---|---|
| A | Water | Water (Aqua) | to 100,0 |
| | Glycerin | Glycerin | 4,00 |
| | Trisodium Ethylenediamine Disuccinate | Trisodium Ethylenediamine Disuccinate | 0,3 |
| B | Xanthan gum | Xanthan gum | 0,1 |
| C | MiyoAQUA White TSR (Miyoshi America) | CI 77891, Aluminum Hydroxide, Algin | 7,9 |
| | MiyoAQUA YELLOW (Miyoshi America) | CI 77492, Algin | 1,35 |
| | MiyoAQUA RED (Miyoshi America) | CI 77491, Algin | 0,55 |
| | MiyoAQUA BLACK NH (Miyoshi America) | CI 77499, Algin | 0,2 |
| D | Plantasens Carefeel Fresh | Limnanthes Alba Seed Oil, Butyrospermum Parkii Butter | 6,0 |
| | Plantasens Olive LD SP ECO | Coco-Caprylate/Caprate, Hydrogenated Olive Oil Unsaponifiables | 7,2 |
| | Plantasens Carefeel Light | Ethylhexyl Olivate | 6,4 |
| | Nipanox BHT | BHT | 0,05 |
| | Plantasens Emulsifier HP10 | Sucrose Polystearate, Cetearyl Alcohol, Olea Europaea Oil Unsaponifiables | 5,0 |
| | Plantasens Emulsifier SFO | Sunflower Seed Oil Sorbitol Esters | 1,0 |
| | Stearic Acid | Stearic acid (vegetal) | 0,75 |
| E | Aristoflex Silk | Sodium Polyacryloyldimethyl Taurate | 0,15 |
| F | Phenoxethanol | Phenoxyethanol | 0,85 |
| | Velsan CGE | Caprylyl Glyceryl Ether | 0,25 |
| | Parfum Nude Rose AFM | Fragrance | 0,3 |
| G | Water | Water (Aqua) | 2,0 |
| | Lactic Acid aqueous solution 80% | Lactic acid | 0,05 |
| | Neutrotain DMG | Dimethylglucamine | 0,15 |
| H | Varrier | Butylene Glycol, Aqua, Morus Nigra Fruit Extract, Lycium Chinense Fruit Extract, Fragaria Fragaria Chiloensis (Strawberry) Fruit Extract, Berberis Vulgaris Root Extract | 2,0 |
| | RedSnow | Propanediol, Aqua, Camellia Japonica Flower Extract | 2,0 |

### Example 21: Eyeshadow

| Phase | Ingredient | INCI | [%] |
|---|---|---|---|
| A | Water | Water (Aqua) | to 100.00 |
| | Glycerin | Glycerin | 3.00 |
| A1 | Aristoflex Silk | Sodium Polyacryloyldimethyl Taurate | 1.00 |
| B | Hostaphat KL 340 D | Trilaureth- 4 Phosphate | 0.50 |
| | Plantasens VP R15 | Ricinus Communis Seed Oil, Hydrogenated Castor Oil, Copernicia Cerifera Wax | 2.00 |
| | Dongbaek Oil | Camellia Japonica Seed Oil | 4.00 |
| | Phenoxetol | Phenoxyethanol | 0.80 |
| | Velsan CGE | Caprylyl Glyceryl Ether | 0.30 |
| | Tocopherol | Tocopherol | 0.50 |
| C | Ronaflair LF-200 | Bismuth Oxychloride, CI 77163 | 5.00 |
| D | Colorona Imperial Topaz | Bismuth Oxychloride, MICA, Iron Oxides | 6.00 |
| E | Orgasol Green Touch | Nylon-11 | 3.00 |
| F | Colorona Bronze Sparkle | MICA, Iron Oxides | 7.00 |
| G | Ronastar Copper Jewel | Calcium Aluminum Borosilicate, CI 77891, Silica, Iron Oxides, Tin Oxide | 4.50 |
| H | Emortal Pep | Propanediol, Aqua, Pisum Sativum Peptide | 1.00 |
| I | B-Circadin | Propanediol, Aqua, Lespedeza Capitata Leaf/stem extract | 2.00 |
| L | NeutroTain DMG | Dimethylglucamine | 0.30 |
| M | Lactic acid 80% | Lactic acid 80% | 0.20 |

### Example 22: Laundry compositions (all% active matter)

| Ingredient | Liquid | Gel / concentrate | Pods / Monodose in water soluble films | Tabs & Powder | Pre-treater | Fabric Softener |
|---|---|---|---|---|---|---|
| Linear Alkylbenzene Sulfonate | | 30 | 25 | 10 | 5 | |
| Sodium Lauryl Ethersulfate | 6 | | 15 | | | |
| Sodium Lauryl Sulfate | | | | 2 | | |
| Laureth-7 | 3 | 30 | 10 | 5 | 8 | |
| Sunfloweroly Glucamide | 3 | | | | | |
| Fatty Acid | 2 | 7 | 3 | | | 2 |
| TEA-Esterquat | | | | | | 6 |
| Solvents | | 25 | Add 100 | | | 1 |
| Enzymes | 2 | 3.5 | 3 | 1 | | |
| Sodium Citrate | 4 | | 1 | | | |
| Soil release polymer | 0-1.5 | 0-1.5 | 0-1.5 | 0-1.5 | 0-1.5 | 0-1.5 |
| Complexing agent | 2 | 3 | 2 | 5 | 0.5 | 0.5 |
| Bleach activator | | | | 8 | | |
| Bleach | | | | 15 | 5 | |
| Builder | | | | 40 | | |
| Filler | | | | Add 100 | | |
| Water | Ad 100 | Ad 100 | 10 | | Ad 100 | Ad 100 |

The compositions may contain perfume and preservatives, as desired / needed.

### Example 23: Automatic dishwashing compositions (all% active matter)

| Ingredient | Rinse Aid | Powder or Tablet (can be in water soluble film) | Pouch / Monodose in water soluble films | Gel | Machine cleaner |
|---|---|---|---|---|---|
| C12/14 Fatty Alcohol EO PO | 9 | 4 | 10 | 1 | |
| Fatty Alcohol Ethoxylate | | | | | 3 |
| Polymers | | 5 | 5 | 10 | |
| Sodium Citrate | 1 | 10 | | 15 | |
| Citric Acid | | | | | 5 |
| Sodium Carbonate | | 35 | 10 | | |
| Silicates | | 15 | | | |
| Bleach activator | | 10 | 8 | | |
| Bleach | | 15 | 15 | | |
| Sodium Cumene Sulfonate | 2 | | | | |
| Solvents | | | 26 | 1 | |
| Enzymes | | 1 | 1 | 2,5 | |
| Complexing agent | 1 | 5 | 25 | 7 | 1 |
| Water | Add 100 | | Add 100 | Ad 100 | Add 100 |

The compositions may contain perfume and preservatives, as desired / needed.

The compositions may further contain, e.g., glass or metal ware protectors.

### Example 24: Hard surface cleaners (especially alkaline products) (all% active matter)

| Ingredient | Kitchen Cleaner | All Purpose Cleaner |
|---|---|---|
| Linear Alkylbenzene Sulfonate | 2 | 1 |
| Sodium Lauryl Ethersulfate | | 2 |
| Fatty Alcohol Ethoxylate | 2 | 0.5 |
| Ethanol | 2 | |
| Complexing Agent | 4 | 1 |
| Water | Add 100 | Ad 100 |

The compositions may contain perfume and preservatives, as desired / needed.

## Claims

1. Use of a sugar amine selected from N,N-dimethylglucamine, N-methylglucamine, N-methylxylamine, N,N-dimethylxylamine, N-methylmaltosamine, N,N-dimethylmaltosamine, N-ethylglucamine, N,N-diethylglucamine, N-methylpropylglucamine, N-2-hydroxyethylglucamine, N,N-methyl 2-hydroxyethylglucamine, N-methylfructosamine, N,N-dimethylfructosamine, N-methylmaltulosamine, N,N-dimethylmaltulosamine, N-methylmaltotriosamine, N,N-dimethylmaltotriosamine, and mixtures thereof, or a salt thereof in a cosmetic composition or a home care composition as a complexing agent, preferably as a chelating agent.

2. Use according to claim 1, wherein the sugar amine is selected from N,N-dimethylglucamine, N-methylglucamine, N-methylxylamine, N-methylmaltosamine, and mixtures thereof.

3. Use according to claim 1 or 2, wherein the sugar amine is N,N-dimethylglucamine.

4. Use according to any of claims 1 to 3, wherein the cosmetic composition is a skin care composition, hair care composition, scalp care composition or oral care composition, preferably wherein the cosmetic composition is selected from liquid soaps, body washes, soap bars, creams, lotions, make up, wet wipes, deodorants, antiperspirants, shampoos, conditioners, coloring shampoos, hair dyes, and toothpaste.

5. Use according to any of claims 1 to 4, wherein the sugar amine or salt thereof is used in a cosmetic composition to increase the stability of the cosmetic composition, to provide or boost preservation, to prevent color degradation, to prevent fragrance degradation, to prevent rancidity, to prevent softening, to prevent brown-spotting, to prevent cracking, to prevent discoloration, to enhance foaming, to enhance rinsability, to prevent off-odors, to prevent the degradation of active ingredients that may be present in the cosmetic composition, to increase the shelf life of the cosmetic composition, to increase the efficacy of vitamins, essential oils or fatty acids that may be present in the cosmetic composition, to provide antioxidant protection, to prevent haze formation and precipitation, to boost antimicrobial agents, to preserve the stability of the cosmetic composition, to stabilize tint and color intensity, or to prevent the formation of reactive oxygen species (ROS).

6. Use according to any of claims 1 to 5, wherein the home care composition is selected from automatic dishwashing products, hand dishwashing detergents, laundry detergents, fabric softeners, and surface cleaners.

7. Use according to any of claims 1 to 6, wherein the sugar amine or salt thereof is used in a home care composition to remove stain, to remove grease, to increase the stability of the home care composition, to provide or boost preservation, to prevent color degradation, or to prevent fragrance degradation.

8. Use according to any of claims 1 to 7, wherein the sugar amine or salt thereof is used as a complexing agent, preferably as a chelating agent, to bind metal ions.

9. Use according to claim 8, wherein the metal ions are magnesium ions, calcium ions, iron ions, copper ions, aluminum ions, zirconium ions, zinc ions, silver ions, titanium ions, or mixtures thereof.

10. Use of a sugar amine selected from N,N-dimethylglucamine, N-methylglucamine, N-methylxylamine, N,N-dimethylxylamine, N-methylmaltosamine, N,N-dimethylmaltosamine, N-ethylglucamine, N,N-diethylglucamine, N-methylpropylglucamine, N-2-hydroxyethylglucamine, N,N-methyl 2-hydroxyethylglucamine, and mixtures thereof, N-methylfructosamine, N,N-dimethylfructosamine, N-methylmaltulosamine, N,N-dimethylmaltulosamine, N-methylmaltotriosamine, N,N-dimethylmaltotriosamine, and mixtures thereof, or a salt thereof as a replacement of ethylenediaminetetraacetic acid (EDTA) or a salt thereof in a cosmetic composition or a home care composition.

11. Use according to claim 10, wherein the sugar amine is selected from N,N-dimethylglucamine, N-methylglucamine, N-methylxylamine, N-methylmaltosamine, and mixtures thereof.

12. Use according to claim 10 or 11, wherein the sugar amine is N,N-dimethylglucamine.

## Patentansprüche

1. Verwendung eines Zuckeramins, das aus N,N-Dimethylglucamin, N-Methylglucamin, N-Methylxylamin, N,N-Dimethylxylamin, N-Methylmaltosamin, N,N-Dimethylmaltosamin, N-Ethylglucamin, N,N-Diethylglucamin, N-Methylpropylglucamin, N-2-Hydroxyethylglucamin, N,N-Methyl-2-hydroxyethylglucamin, N-Methylfructosamin, N,N-Dimethylfructosamin, N-Methylmaltulosamin, N,N-Dimethylmaltulosamin, N-Methylmaltotriosamin, N,N-Dimethylmaltotriosamin und Mischungen davon ausgewählt ist, oder eines Salzes davon in einer kosmetischen Zusammensetzung oder einer Haushaltspflegezusammensetzung als Komplexbildner, vorzugsweise als Chelatbildner.

2. Verwendung nach Anspruch 1, wobei das Zuckeramin aus N,N-Dimethylglucamin, N-Methylglucamin, N-Methylxylamin, N-Methylmaltosamin und Mischungen davon ausgewählt ist.

3. Verwendung nach Anspruch 1 oder 2, wobei es sich bei dem Zuckeramin um N,N-Dimethylglucamin handelt.

4. Verwendung nach einem der Ansprüche 1 bis 3, wobei es sich bei der kosmetischen Zusammensetzung um eine Hautpflegezusammensetzung, Haarpflegezusammensetzung, Kopfhautpflegezusammensetzung oder Mundpflegezusammensetzung handelt, wobei die kosmetische Zusammensetzung vorzugsweise aus Flüssigseifen, Duschgelen, Stückseifen, Cremes, Lotionen, Make-up, Feuchttüchern, Deodorantien, Antitranspirantien, Shampoos, Konditionierungsmitteln, Färbeshampoos, Haarfärbemitteln und Zahnpasta ausgewählt ist.

5. Verwendung nach einem der Ansprüche 1 bis 4, wobei das Zuckeramin oder Salz davon in einer kosmetischen Zusammensetzung verwendet wird, um die Stabilität der kosmetischen Zusammensetzung zu erhöhen, um Konservierung bereitzustellen oder zu erhöhen, um Farbabbau zu verhindern, um Duftstoffabbau zu verhindern, um Ranzigkeit zu verhindern, um Erweichung zu verhindern, um die Bildung von braunen Flecken zu verhindern, um Rissbildung zu verhindern, um Verfärbung zu verhindern, um das Schäumen zu verbessern, um die Ausspülbarkeit zu verbessern, um Fehlgerüche zu verhindern, um den Abbau von Wirkstoffen, die in der kosmetischen Zusammensetzung vorhanden sein können, zu verhindern, um die Haltbarkeit der kosmetischen Zusammensetzung zu erhöhen, um die Wirksamkeit von Vitaminen, ätherischen Ölen oder Fettsäuren, die in der kosmetischen Zusammensetzung vorhanden sein können, zu erhöhen, um einen antioxidativen Schutz bereitzustellen, um Trübungsbildung und Ausfällung zu verhindern, um antimikrobielle Mittel zu verstärken, um die Stabilität der kosmetischen Zusammensetzung zu erhalten, um Farbton und Farbintensität zu verbessern oder um die Bildung reaktiver Sauerstoffspezies (ROS) zu verhindern.

6. Verwendung nach einem der Ansprüche 1 bis 5, wobei die Haushaltspflegezusammensetzung aus Produkten für das automatische Geschirrspülen, Handgeschirrspülmitteln, Waschmitteln, Weichspülern und Oberflächenreinigern ausgewählt ist.

7. Verwendung nach einem der Ansprüche 1 bis 6, wobei das Zuckeramin oder Salz davon in einer Haushaltspflegezusammensetzung verwendet wird, um Flecken zu entfernen, um Fett zu entfernen, um die Stabilität der Haushaltspflegezusammensetzung zu erhöhen, um Konservierung bereitzustellen oder zu verstärken, um Farbabbau zu verhindern oder um Duftstoffabbau zu verhindern.

8. Verwendung nach einem der Ansprüche 1 bis 7, wobei das Zuckeramin oder Salz davon als Komplexbildner, vorzugsweise als Chelatbildner, zum Binden von Metallionen verwendet wird.

9. Verwendung nach Anspruch 8, wobei es sich bei den Metallionen um Magnesiumionen, Calciumionen, Eisenionen, Kupferionen, Aluminiumionen, Zirconiumionen, Zinkionen, Silberionen, Titanionen oder Mischungen davon handelt.

10. Verwendung eines Zuckeramins, das aus N,N-Dimethylglucamin, N-Methylglucamin, N-Methylxylamin, N,N-Dimethylxylamin, N-Methylmaltosamin, N,N-Dimethylmaltosamin, N-Ethylglucamin, N,N-Diethylglucamin, N-Methylpropylglucamin, N-2-Hydroxyethylglucamin, N,N-Methyl-2-hydroxyethylglucamin und Mischungen davon, N-Methylfructosamin, N,N-Dimethylfructosamin, N-Methylmaltulosamin, N,N-Dimethylmaltulosamin, N-Methylmaltotriosamin, N,N-Dimethylmaltotriosamin und Mischungen davon ausgewählt ist, oder eines Salzes davon als Ersatz für Ethylendiaminotetraessigsäure (EDTA) oder ein Salz davon in einer kosmetischen Zusammensetzung oder einer Haushaltspflegezusammensetzung.

11. Verwendung nach Anspruch 10, wobei das Zuckeramin aus N,N-Dimethylglucamin, N-Methylglucamin, N-Methylxylamin, N-Methylmaltosamin und Mischungen davon ausgewählt ist.

12. Verwendung nach Anspruch 10 oder 11, wobei es sich bei dem Zuckeramin um N,N-Dimethylglucamin handelt.

## Revendications

1. Utilisation d'une amine de sucre choisie parmi la N,N-diméthylglucamine, la N-méthylglucamine, la N-méthylxylamine, la N,N-diméthylxylamine, la N-méthylmaltosamine, la N,N-diméthylmaltosamine, la N-éthylglucamine, la N,N-diéthylglucamine, la N-méthylpropylglucamine, la N-2-hydroxyéthylglucamine, la N,N-méthyl 2-hydroxyéthylglucamine, la N-méthyl fructosamine, la N,N-diméthylfructosamine, la N-méthylmaltulosamine, la N,N-diméthylmaltulosamine, la N-méthylmaltotriosamine, la N,N-diméthylmaltotriosamine, et leurs mélanges, ou d'un sel de celles-ci dans une composition cosmétique ou une composition d'entretien ménager comme agent complexant, de préférence comme agent chélatant.

2. Utilisation selon la revendication 1, dans laquelle l'amine de sucre est choisie parmi la N,N-diméthylglucamine, la N-méthylglucamine, la N-méthylxylamine, la N-méthylmaltosamine et leurs mélanges.

3. Utilisation selon la revendication 1 ou 2, dans laquelle l'amine de sucre est la N,N-diméthylglucamine.

4. Utilisation selon l'une quelconque des revendications 1 à 3, dans laquelle la composition cosmétique est une composition de soin de la peau, une composition de soin des cheveux, une composition de soin du cuir chevelu ou une composition de soin buccal, de préférence dans laquelle la composition cosmétique est choisie parmi les savons liquides, les nettoyants corporels, les savons en barre, les crèmes, les lotions, le maquillage, les lingettes humides, les déodorants, les antitranspirants, les shampooings, les après-shampooings, les shampooings colorants, les teintures capillaires et un dentifrice.

5. Utilisation selon l'une quelconque des revendications 1 à **4,** dans laquelle l'amine de sucre ou un sel de celle-ci est utilisé dans une composition cosmétique pour augmenter la stabilité de la composition cosmétique, pour assurer ou renforcer la conservation, pour prévenir la dégradation de la couleur, pour prévenir la dégradation du parfum, pour prévenir le rancissement, pour prévenir le ramollissement, pour prévenir les taches brunes, pour prévenir les craquelures, pour prévenir la décoloration, pour améliorer la formation de mousse, pour améliorer la rinçabilité, pour prévenir les mauvaises odeurs, pour prévenir la dégradation des ingrédients actifs qui peuvent être présents dans la composition cosmétique, pour augmenter la durée de conservation de la composition cosmétique, pour augmenter l'efficacité des vitamines, des huiles essentielles ou des acides gras qui peuvent être présents dans la composition cosmétique, pour fournir une protection antioxydante, pour prévenir la formation de trouble et la précipitation, pour renforcer les agents antimicrobiens, pour préserver la stabilité de la composition cosmétique, pour stabiliser la teinte et l'intensité de la couleur, ou pour prévenir la formation d'espèces réactives de l'oxygène (ROS).

6. Utilisation selon l'une quelconque des revendications 1 à 5, dans laquelle la composition d'entretien ménager est choisie parmi les produits pour lave-vaisselle automatique, les détergents pour lave-vaisselle à la main, les détergents pour lessive, les assouplissants pour tissus et les nettoyants de surface.

7. Utilisation selon l'une quelconque des revendications 1 à 6, dans laquelle l'amine de sucre ou un sel de celle-ci est utilisé dans une composition d'entretien ménager pour éliminer les taches, pour éliminer la graisse, pour augmenter la stabilité de la composition d'entretien ménager, pour assurer ou renforcer la conservation, pour prévenir la dégradation de la couleur ou pour prévenir la dégradation du parfum.

8. Utilisation selon l'une quelconque des revendications 1 à 7, dans laquelle l'amine de sucre ou un sel de celle-ci est utilisé comme agent complexant, de préférence comme agent chélatant, pour lier des ions métalliques.

9. Utilisation selon la revendication 8, dans laquelle les ions métalliques sont des ions magnésium, des ions calcium, des ions fer, des ions cuivre, des ions aluminium, des ions zirconium, des ions zinc, des ions argent, des ions titane ou leurs mélanges.

10. Utilisation d'une amine de sucre choisie parmi la N,N-diméthylglucamine, la N-méthylglucamine, la N-méthylxylamine, la N,N-diméthylxylamine, la N-méthylmaltosamine, la N,N-diméthylmaltosamine, la N-éthylglucamine, la N,N-diéthylglucamine, la N-méthylpropylglucamine, la N-2-hydroxyéthylglucamine, la N,N-méthyl 2-hydroxyéthylglucamine, et leurs mélanges, la N-méthylfructosamine, la N,N-diméthylfructosamine, la N-méthylmaltulosamine, la N,N-diméthylmaltulosamine, la N-méthylmaltotriosamine, la N,N-diméthylmaltotriosamine, et leurs mélanges, ou d'un sel de celles-ci en remplacement de l'acide éthylènediaminetétraacétique (EDTA) ou d'un sel de celui-ci dans une composition cosmétique ou une composition d'entretien ménager.

11. Utilisation selon la revendication 10, dans laquelle l'amine de sucre est choisie parmi la N,N-diméthylglucamine, la N-méthylglucamine, la N-méthylxylamine, la N-méthylmaltosamine et leurs mélanges.

12. Utilisation selon la revendication 10 ou 11, dans laquelle l'amine de sucre est la N,N-diméthylglucamine.
